# EUROPEAN PATENT SPECIFICATION

(11) **EP 1 650 186 B1**
(45) Date of publication and mention of the grant of the patent: **04.06.2008**
(21) Application number: 04292517.2
(22) Date of filing: 22.10.2004
(51) Int. Cl.: C07C 229/30, C07C 229/48, C07C 237/24, C07C 323/12, A61K 31/10, A61K 31/165, A61K 31/196, A61K 31/197, A61K 31/216, A61P 37/06

(54) **Novel dicarboxylic acid derivatives**
Neue Dicarbonsäurederivate
Nouveaux derivés d'acides dicarboxyliques

(43) Date of publication of application: 26.04.2006
(73) Proprietor: BIOPROJET, 75003 Paris (FR); Sun Pharmaceutical Industries Ltd, Akota, Baroda 390020 Gujarat (IN)
(72) Inventor: Capet, Marc, 35520 Melesse (FR); Levoin, Nicolas, 35310 Mordelles (FR); Berrebi-Bertrand, Isabelle, 35740 Pace (FR); Poupardin, Olivia, 35830 Betton (FR); Robert, Philippe, 35740 Pace (FR); Schwartz, Jean-Charles, 75014 Paris (FR); Lecomte, Jeanne-Marie, 75003 Paris (FR); Rajamannar, Thennati, Baroda 390 015 (IN); Pal, Ranjan Kumar A/4 Shreeji Park Duplex, New Sama Road, Baroda 390008 (IN); Samanta, Biswajit, Baroda 390 015 (IN); Jivani, Jignesh K, Baroda 390 002 (IN); Panchal, Bhavesh M., Vallabh Vidyanagar 388120 (IN); Bhatt, Isha H. A/3, Satkar Society, Baroda 390018 (IN); Aradhye, Jayraj D., Baroda 390018 (IN)
(74) Representative: Colombet, Alain André

(56) References cited:
- EP-A- 0 063 481
- WO-A-20/04028521
- GB-A- 917 817
- US-A- 3 325 360
- US-A- 5 480 908
- US-A- 5 482 971
- US-A- 5 488 064
- US-A- 5 914 339
- DATABASE CA [Online] CHEMICAL ABSTRACTS SERVICE, COLUMBUS, OHIO, US; 1996, FUJITA, MASAHIRO ET AL: "Preparation and formulation of quinolinecarboxylic acid and naphthyridinecarboxylic acid derivatives as medical bactericides" XP002325951 retrieved from STN Database accession no. 1996:259448 -& WO 96/01260 A1 (DAINIPPON PHARMACEUTICAL CO., LTD., JAPAN) 18 January 1996 (1996-01-18)
- CURTIUS T ET AL: "DER ZERFALL DES BENZYLAZIDS IN INDIFFERENTEN MEDIEN UND IN MALONESTER" CHEMISCHE BERICHTE, vol. 55, 1922, pages 1559-1571, XP009046735 ISSN: 0009-2940
- MAIER L ET AL: "ORGANIC PHOSPHORUS COMPOUNDS 761 SYNTHESIS AND PROPERTIES OF PHOSPHINOTHRICIN DERIVATIVES" PHOSPHORUS SULFUR AND THE RELATED ELEMENTS, GORDON AND BREACH - HARWOOD ACADEMIC, CH, vol. 17, 1983, pages 1-20, XP009046741 ISSN: 0308-664X
- SIMIG G ET AL: "SIMPLE AND CONDENSED BETA-LACTAMS-I THE APPLICATION OF DIKETENE IN BETA-LACTAM SYNTHESIS. THE SYNTHESIS AND FUNCTIONAL GROUP MANIPULATIONS OF DIETHYL 3-ACETYL-4-OXOAZETIDINE-2,2-DICARBOXYLATES " TETRAHEDRON, ELSEVIER SCIENCE PUBLISHERS, AMSTERDAM, NL, vol. 41, no. 2, 1985, pages 479-484, XP001206149 ISSN: 0040-4020
- SIMIG G ET AL: "SIMPLE AND CONDENSED BETA-LACTAMS, II THE SYNTHESIS OF NEW DIETHYL 4-OXOAZETIDINE-2,2-DICARBOXYLATES AND SOME MANIPULATIONS OF THEIR FUNCTIONAL GROUPS AND N-SUBSTITUENTS" ACTA CHIMICA HUNGARICA, AKADEMIAI KIADO, BUDAPEST, HU, vol. 119, no. 1, 1985, pages 17-32, XP009046747 ISSN: 0231-3146
- HUSINEC S ET AL: "BIS(METHOXYCARBONYL)CARBENE INSERTION INTO N-H BONDS: A FACILE ROUTE TO N-SUBSTITUTED AMINOMALONIC ESTERS" SYNTHESIS, THIEME, STUTTGART, DE, vol. 9, September 1988 (1988-09), pages 721-723, XP001206244 ISSN: 0039-7881
- KNOELKER H-J ET AL: "TRANSITION METAL COMPLEXES IN ORGANIC SYNTHESIS, PART 72: IRON-MEDIATED DIASTEREOSELECTIVE SPIROANNELATION OF DIMETHYL AMINOMALONATE TO THE 2-AZASPIROÄ4.5ÜDECANE RING SYSTEM" SYNLETT, THIEME VERLAG, STUTTGART, DE, vol. 10, 2004, pages 1769-1771, XP001206147 ISSN: 0936-5214
- HAYAKAWA T ET AL: "THE SYNTHESIS OF OPTICALLY-ACTIVE VALINE BY THE STEREOSELECTIVE DECARBOXYLATION OF ALPHA(ALPHA-METHYLBENZYLAMINO)-ALPHA-ISOPR OPYLMAL ONIC ACID" BULLETIN OF THE CHEMICAL SOCIETY OF JAPAN, NIHON KAGAKKAI, TOKYO, JP, vol. 46, no. 6, 1973, pages 1886-1887, XP009046845 ISSN: 0009-2673
- SUGASAWA S ET AL: "STUDIES ON THE SYNTHESIS OF EMETINE. I. A SYNTHESIS OF 1-(N-BENZYL-2-OXO-5-ETHYL-4-PIPERIDYLMETHY L)-6,7-DIMETHOXY-3,4-DIHYDR OISOQUINOLINE. (PRELIMINARY EXPERIMENTS TO THE SYNTHESIS OF EMETINE)" PHARMACEUTICAL BULLETIN, PHARMACEUTICAL SOCIETY OF JAPAN, TOKYO,, JP, vol. 3, no. 1, 1955, pages 47-52, XP009046756 ISSN: 0369-9471
- JENHI A ET AL: "2,5-IMIDAZOLIDINEDIONE FORMATION FROM SEVEN MEMBERED CYCLODIPEPTIDE REARRANGEMENT" SYNTHETIC COMMUNICATIONS, MARCEL DEKKER, NEW YORK, NY, US, vol. 31, no. 11, 2001, pages 1707-1714, XP009046842 ISSN: 0039-7911
- GITTOS M W ET AL: "INTRAMOLECULAR INTERACTION BETWEEN GAMMA-TERTIARY AMINO- AND CYANO-GROUPS" JOURNAL OF THE CHEMICAL SOCIETY, CHEMICAL SOCIETY. LETCHWORTH, GB, 1955, pages 2371-2376, XP009046757 ISSN: 0368-1769
- OSBOND J M: "CHEMICAL CONSTITUTION AND AMOEBICIDAL ACTIVITY. PART VI. A NEW SYNTHESIS OF 2-KETONES AND 2-ALCOHOLS DERIVED FROM 3-ALKYL-1,3,4,6,7,11B-HEXAHYDRO-9,10-DIMET HOXY-2H-BENZO??UINOLIZINES" JOURNAL OF THE CHEMICAL SOCIETY, CHEMICAL SOCIETY. LETCHWORTH, GB, 1961, pages 4711-4718, XP009046828 ISSN: 0368-1769
- ITOH N: "SYNTHESIS OF 1-SUBSTITUTED 4-PHENYL-4-PIPERIDINOL DERIVATIVES AS POSSIBLE ANALGESIC" CHEMICAL AND PHARMACEUTICAL BULLETIN, TOKYO, JP, vol. 10, 1962, pages 55-60, XP009046833 ISSN: 0009-2363
- HALE J. J. ET AL: "Selecting against S1P3 enhances the acute cardiovascular tolerability of 3-(N-benzyl)aminopropylphosphonic acid S1P receptor agonists" BIOORG. & MED. CHEM. LETT., vol. 14, 2004, pages 3501-3505, XP002325960

## Description

The present patent application concerns new compounds displaying agonistic activity at sphingosine-1-phosphate (S1P) receptors, their process of preparation and their use as immunosuppressive agents.
Among other effects, indirect or direct S1P receptor agonists inhibit thymic egress and lymphocyte recirculation (Rosen et al, Immunol. Rev. 2003, 195, 160).
Inhibition of lymphocyte egress is associated with clinically useful immunosuppression in both transplantation and autoimmune diseases.
Agonism of sphingosine-1-phosphate receptors (particularly S1 P1 receptors) induces accelerated homing of lymphocytes to lymph nodes and Peyer's patches without lymphodepletion. Such immunosuppression is desirable to prevent rejection after organ transplantation and in the treatment of autoimmune disorders.
Immunosuppressive agents have been shown to be useful in a wide variety of autoimmune and chronic inflammatory diseases including transplant rejection, multiple sclerosis, lupus erythematosus, rheumatoid arthritis, inflammatory bowel disease, psoriasis, asthma, myocarditis, atopic dermatitis, type-1 diabetes, atherosclerosis, glomerulonephritis, lymphocytic leukemias, lymphomas, multiorgan failure, sepsis, pneumonia, etc.
This mechanism was recently shown to be operant in the well demonstrated immunosuppression of FTY 720, a drug currently submitted to clinical trials in the prevention of organ transplant rejection (Matioubian et al, Nature, 427, pp. 355-360, 2004). FTY 720 is a synthetic analog of a natural compound derived from the fungus *Isaria sinclairii.* FTY 720 induces a decrease in circulating lymphocytes and the efficacy has been attributed to arise from the agonist driven functional mechanism of S1 P1. This compound advanced to Phase III clinical trials for the treatment of organ transplantation and in Phase II for multiple sclerosis.
However, FTY 720 is reported to have an adverse event of transient asymptomatic bradycardia (J. Am. Soc. Nephrol., 13, 1073, 2002) and the toxicity potential is mechanism based due to non selective agonism on S1 P3 receptor *(*Bioorg. & Med. Chem. Lett., 14, 3501, 2004).
Recently, further selective agonists of S1P have also been described in the patent literature (WO 04 058149, WO 04 024673, WO 03 061567).
However, FTY 720 and congeners suffer from two potential drawbacks in this indication: they require to be phosphorylated *in vivo* to become active (Brinkmann et al, J. Biol. Chem., 277, 24, pp. 21453-21457, 2002) and suffer from a lack of selectivity towards the five S1P receptor subtypes (Mandala et al, Science, 296, pp. 346-349, 2002).
There is therefore potential interest in developing direct S1P receptor agonists displaying receptor selectivity, particularly compounds with low relative activity at the S1P3 receptor subtype expressed in cardiac tissues and whose activation results in bradycardia and cardiac depression (Forrest et al, JPET, 309, pp. 758-768, 2004, Sanna et al, J. Biol. Chem., 279 (14), pp. 13839-13848, 2004)
US 5,480,908**,** US 5,482,971**,** US 5,914,339 **and** US 5,488,064 **disclose 1,3-benzodioxole dicarboxylate derivatives and their use as β3-adrenergic receptor agonists.**
It is thus an object of the present invention to provide novel compounds that are S1P receptor agonists, having immunosuppressive activity preferably with low affinity to edg3/S1P3 receptor.
Surprisingly, the present inventors have identified new compounds of formula (I) which fulfill these requirements.

According to a first object, the present invention concerns new compounds of formula (I): 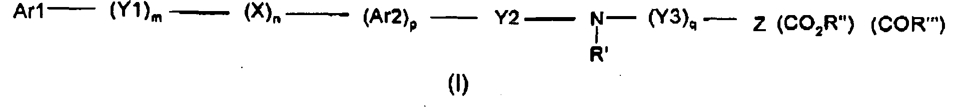
- Ar1 represents an Aryl group optionally substituted by 1 up to 5 R group(s),
wherein, each R, identical or different, is selected from the group consisting in Halogen atom, -Alkyl, -OAlkyl, -OH, -COOR7, -CONR7R8, -Alkyl-Hal, -OAlkyl-Hal, NO₂, -CN, -NR7R8, -AlkylAryl, -Aryl, -S(O)ₗR7, -Alkenyl, -Si(Alkyl)₃;
wherein R7 and R8, identical or different, are chosen from the group consisting in H ; Alkyl optionally substituted by a Halogen atom, polyfluoroalkyl, Aryl; Heteroaryl; or R7 and R8 form together with the N atom to which they are attached a Heterocycle;
■ l is O, 1 or 2;
■ Y1 represents an -Alkyl- chain, optionally substituted by one or more R as defined above, and optionally comprising one or more insaturation(s) and/or heteroatom(s) and/or a residue chosen from the group consisting in -Alken-, -Alkyn-, -CH=N-, -N=CH-, -CH=N-O-, -O-N=CH-, -C(=O)-, -C(=O)(OR7)-, -C(=O)(NR7)-, -N=N-, -S(O)_{I}- :
■ m is 0 or 1 ;
■ X represents a heteroatom;
■ n is 0 or 1;
■ -Ar2- represents an -Aryl- group optionally substituted by one or more R group as defined above or wherein 2 R may form together with the atoms to which they are attached a fused cyclic, aryl or heteroaryl ring :
■ p is 0 or 1;
■ -Y2- represents an -Alkyl- chain, optionally substituted by one or more R as defined above, and optionally comprising one or more insaturation(s) or heteroatom(s) and/or a residue chosen from the group consisting in -Alken-, -Alkyn-, -CH=N-, -N=CH-, -CH=N-O-, -O-N=CH-, -C(=O)-, -C(=O)O-, -C(=O)(NR7)--N=N-, -S(O)ₗ- :
-R' represents a hydrogen atom, or an -Alkyl chain, optionally substituted by one or more R as defined above, and optionally comprising one or more insaturation(s) or heteroatom(s) and/or a residue chosen from the group consisting in -Alken-, -Alkyn-, -CH=N-, -N=CH-, -CH=N-O-, -O-N=CH-, -C(=O)O-, -C(=O)(NR7)-, -N=N-, -S(O)ₗ-;
■ -Y3- represents an -Alkyl- chain, optionally substituted by one or more R as defined above, and optionally comprising one or more insaturation(s) or heteroatom(s) and/or a residue chosen from the group consisting in -Alken-, -Alkyn-, -CH=N-, -N=CH-, -CH=N-O-, -O-N=CH-, -C(=O)-, -C(=O)O-, -C(=O)(NR7), -N=N-, -S(O)ₗ-;
• q is 0 or 1;
• Z represents a -Cycloalkyl< group or -Aryl<, or -C(alkyl)< group, or R' and Z form together with the N atom to which they are attached a Heterocyclic or Heteroaryl group; wherein (CO₂R'') and (COR''') can be attached to the same atom or two adjacent atoms ;
■ -R" represents a hydrogen atom or an Alkyl chain, optionally substituted by one or more R as defined above, and optionally comprising one or more insaturation(s) ;
■ -R''' represents -OH, -OAlkyl, H, -NR7OR8, -NR7R8, natural or synthetic amino acids, wherein R7 and R8 are defined as above;
or R" and R''' form together a ring with the atom(s) to which they are attached to form a cyclic intramolecular bis carbonyl group, including Meldrum acid derivatives;
as well as their enantiomers, diastereoisomers, mixtures thereof and pharmaceutically acceptable salts, hydrates, solvates
with the exception of compounds where :
- m=n=p=0, q=0 or 1, R'=H or Alkyl, Y2=-Alkyl-, Y3=-Alkyl-, Z=-C(Afkyl)< and Ar1= unsubstituted phenyl or phenyl substituted with one or more identical R where R is Halogen; and
- m=n=p=0, q= 1, R'=H, Y2=-CH₂-, Y3=-CH₂-, Z=-cyctobutyl< and Ar1= unsubstituted phenyl.

Preferably, the compounds of the invention are represented by the following general formula (II): 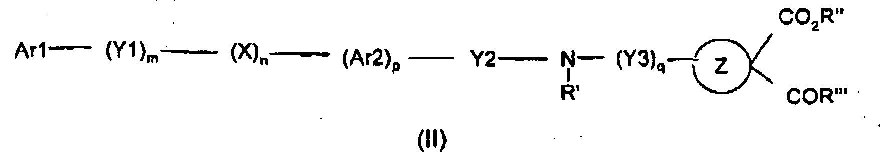 in which Ar1, Y1, X, Ar2, Y2, R, R', Y3, R", R"', m, n, p, q are defined as above and 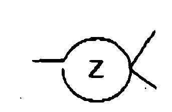 represents a -Cycloalkyl< group or an -Aryl<,
wherein (CO₂R") and (COR"') can be attached to the same atom or two adjacent atoms of the Z ring ;
as well as their enantiomers, diastereoisomers, mixtures thereof and pharmaceutically acceptable salts, hydrates, solvates.

Preferably, in the general formula (II) :
■ Ar1 represents an Aryl group optionally substituted, preferably substituted, by 1 up to 5 R group(s),
   wherein, each R, identical or different, is selected from the group consisting in Halogen atom, -Alkyl, -OAlkyl,
■ Y1 represents an -Alkyl- chain or a -S-Alkyl- chain;
■ m is 0 or 1;
■ X represents a heteroatom;
■ n is 0 or 1;
■ -Ar2- represents an -Aryl- group:
■ p is 0 or 1;
■ -Y2- represents an -Alkyl- chain;
■ -R' represents a hydrogen atom or an alkyl chain;
■ q is 0;
■ 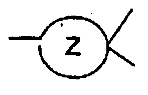 represents a -Cycloalkyl< group and (CO₂R") and (COR"') are attached to the same atom of the Z ring;
■ -R" represents a hydrogen atom;
■ -R"' represents -OH, -OAlkyl, -NR7R8.

In the general formula (I) or (II):

Preferably, Ar1 represents a Phenyl group optionally substituted, preferably substituted, by 1 up to 5 R group(s),
wherein, each R₁ identical or different, is selected from the group consisting in Halogen atom, -Alkyl, -OAlkyl,
Preferably, Y1 represents an Alkyl- chain or a -S-Alkyl- chain;
Preferably, m is 0 or 1;
Preferably, X represents an Oxygen atom;
Preferably, n is 0 or 1;
Preferably, -Ar2- represents an -Phenyl- group;
Preferably, p is 0 or 1;
Preferably, -Y2- represents an -Alkyl- chain;
Preferably, -R' represents a hydrogen atom;
Preferably, q is 0 or 1;
Preferably, Y3 represents an alkynyl chain;

Preferably, Z represents a -C(alkyl)< group; or 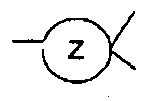 represents a -Cycloalkyl< group, wherein (CO₂R") and (COR"') are attached to the same atom of the Z ring ;
Preferably, -R" represents a hydrogen atom;
Preferably, -R'" represents -OH, -OAlkyl, -NR7R8

According to a preferred aspect, compounds of the invention are selected from the group consisting in:
3-(4-octylbenzylamino)cyclopentane-1,1-dicarboxylic acid
3-(4-octylbenzylamino)cyclopentane-1,1-dicarboxylic acid ethyl ester
3-(4-nonylbenzylamino)cyclopentane-1,1-dicarboxylic acid
3-(4-octyloxybenzylamino)cyclopentane-1,1-dicarboxylic acid
3-(3-methoxy-4-octyloxybenzylamino)cyclopentane-1,1-dicarboxylic acid
3-(3-methoxy-4-butyloxybenzylamino)cyclopentane-1,1-dicarboxylic acid
3-(3-methoxy-4-hexyloxybenzylamino)cyclopentane-1,1-dicarboxylic acid
3-(3-bromo-4-octyloxy-5-methoxybenzylamino)cyclopentane-1,1-dicarboxylic acid
3-(3-methoxy-4-octyloxy-5-methylbenzylamino)cyclopentane-1,1-dicarboxylic acid
3-(3-chloro-4-octyloxy-5-methoxybenzylamino)cyclopentane-1,1-dicarboxylic acid
3-(4-nonyloxybenzylamino)cyclopentane-1,1-dicarboxylic acid
3-(4-decylbenzylamino)cyclopentane-1,1-dicarboxylic acid
3-(3-methoxy-4-nonyloxybenzylamino)cyclopentane-1,1-dicarboxylic acid
3-(3-chloro-4-nonyloxybenzylamino)cyclopentane-1,1-dicarboxylic acid
3-[4-[4-(4-methoxyphenyl)butoxy]benzylamino]cyclopentane-1,1-dicarboxylic acid
3-[4-[4-(3-chloro-4-methoxyphenyl)butoxy]benzylamino]-cyclopentane-1,1-dicarboxylic acid
3-(4-nonylbenzylamino)cyclopentane-1,1-dicarboxylic acid ethyl ester hydrochloride
3-{4-[3-fluoro-(4-hexylphenyl)benzyl]amino}cyclopentane-1,1-dicarboxylic acid hydrochloride
1-(2,2,2-trifluoroethylcarbamoyl)-3-(4-nonylbenzylamino)cyclopentanecarboxylic acid hydrochloride (isomer A)
1-(2,2,2-trifluoroethylcarbamoyl)-3-(4-nonylbenzylamino)cyclopentanecarboxylic acid hydrochloride (isomer B)
3-(4-decyloxybenzylamino)cyclopentane-1,1-dicarboxylic acid hydrochloride
1-(methylcarbamoyl)-3-(4-nonylbenzylamino)cyclopentanecarboxylic acid hydrochloride (isomer A)
1-(methylcarbamoyl)-3-(4-nonylbenzylamino)cyclopentanecarboxylic acid hydrochloride (isomer B)
3-(methyl-4-nonylbenzylamino)cyclopentane-1,1-dicarboxylic acid hydrochloride
3-[4-(4-phenylsulfanylbutoxy)benzylamino]cyclopentane-1,1-dicarboxylic acid hydrochloride
3-{4-[4-(4-ethoxy-3-chlorophenyl)butoxy]benzylamino}cyclopentane-1,1-dicarboxylic acid hydrochloride
2-methyl-2-[4-(4-nonylbenzylamino)but-2-ynyl]malonic acid hydrochloride
2-[4-(3-chloro-4-nonylbenzylamino)but-2-ynyl]-2-methylmalonic acid hydrochloride
2-methyl-2-[4-(4-octylbenzylamino)but-2-ynyl]malonic acid hydrochloride
2-[4-(4-decylbenzylamino)but-2-ynyl]-2-methylmalonic acid hydrochloride
2-[3-fluoro-4-(4-hexylphenyl)benzylamino]but-2-ynyl]-2-methylmalonic acid hydrochloride
as well as their enantiomers, diastereoisomers, mixtures thereof, free forms and pharmaceutically acceptable salts, hydrates, solvates.

According to a still preferred aspect, compounds of the invention are selected from the group consisting in:
3-(4-nonylbenzylamino)cyclopentane-1,1-dicarboxylic acid
3-(4-decylbenzylamino)cyclopentane-1,1-dicarboxylic acid
as well as their enantiomers, diastereoisomers, mixtures thereof, free forms and pharmaceutically acceptable salts, hydrates, solvates.

As used hereabove or hereafter:
"Alkyl" means an aliphatic hydrocarbon group which may be straight or branched having 1 to 20 carbon atoms in the chain. Preferred alkyl groups have 1 to 12 carbon atoms in the chain. Branched means that one or more lower alkyl groups such as methyl, ethyl or propyl are attached to a linear alkyl chain. Exemplary alkyl groups include methyl, ethyl, *n*-propyl, *i*-propyl, *n*-butyl, *t*-butyl, *n-*pentyl, 3-pentyl, octyl, nonyl, decyl.
"Alken" means an aliphatic hydrocarbon group containing a carbon-carbon double bond and which may be straight or branched having 2 to 15 carbon atoms in the chain. Preferred alkenyl groups have 2 to 12 carbon atoms in the chain; and more preferably about 2 to 4 carbon atoms in the chain. Exemplary alkenyl groups include ethenyl, propenyl, *n*-butenyl, *i*-butenyl, 3-methylbut-2-enyl, *n*-pentenyl, heptenyl, octenyl, nonenyl, decenyl.
"Alkyn" means an aliphatic hydrocarbon group containing a carbon-carbon triple bond and which may be straight or branched having 2 to 15 carbon atoms in the chain. Preferred alkynyl groups have 2 to 12 carbon atoms in the chain; and more preferably 2 to 4 carbon atoms in the chain. Exemplary alkynyl groups include ethynyl, propynyl, *n*-butynyl, 2-butynyl, 3-methylbutynyl, *n*-pentynyl, heptynyl, octynyl and decynyl.
"Halogen atom" refers to fluorine, chlorine, bromine or iodine atom; preferably fluorine and chlorine atom.
"Cycloalkyl" means a non-aromatic mono- or multicyclic hydrocarbon ring system of 3 to 10 carbon atoms, preferably of 5 to 10 carbon atoms. Preferred ring sizes of rings of the ring system include 5 to 6 ring atoms. Exemplary monocyclic cycloalkyl include cyclopentyl, cyclohexyl, cycloheptyl, and the like. Exemplary multicyclic cycloalkyl include 1-decalin, norbornyl, adamant-(1- or 2-)yl.
"Aryl" means an aromatic monocyclic or multicyclic hydrocarbon ring system of 6 to 14 carbon atoms, preferably of 6 to 10 carbon atoms. Exemplary aryl groups include phenyl or naphthyl.
As used herein, the terms "heterocycle" or "heterocyclic" refer to a saturated, partially unsaturated or unsaturated, non aromatic stable 3 to 14, preferably 5 to 10 membered mono, bi or multicyclic rings wherein at least one member of the ring is a hetero atom. Typically, heteroatoms include, but are not limited to, oxygen, nitrogen, sulfur, selenium, and phosphorus atoms. Preferable heteroatoms are oxygen, nitrogen and sulfur.
Suitable heterocycles are also disclosed in The Handbook of Chemistry and Physics, 76th Edition, CRC Press, Inc., 1995-1996, pages 2-25 to 2-26.
Preferred non aromatic heterocyclic include, but are not limited to oxiranyl, tetrahydrofuranyl, dioxolanyl, tetrahydropyranyl, dioxanyl, pyrrolidinyl, piperidyl, morpholinyl, imidazolidinyl, pyranyl, imidazolinyl, pyrrolinyl, pyrazolinyl.
Preferred aromatic heterocyclic, herein called heteroaryl groups include, but are not limited to, pyridyl, pyridyl-N-oxide, pyrimidinyl, pyrrolyl, furanyl, thienyl, imidazolyl, triazolyl, tetrazolyl, quinolyl, isoquinolyl, benzoimidazolyl, thiazolyl, pyrazolyl, and benzothiazolyl groups.

As used herein, the term "heteroaryl" refers to a 5 to 14, preferably 5 to 10 membered aromatic hetero, mono-, bi- or multicyclic ring. Examples include pyrrolyl, pyridyl, pyrazolyl, thienyl, pyrimidinyl, pyrazinyl, tetrazolyl, indolyl, quinolinyl, purinyl, imidazolyl, thienyl, thiazolyl, benzothiazolyl, furanyl, benzofuranyl, 1,2,4-thiadiazolyl, isothiazolyl, triazoyl, tetrazolyl, isoquinolyl, benzothienyl, isobenzofuryl, pyrazolyl, carbazolyl, benzimidazolyl, isoxazolyl.
"Alkyl", "cycloalkyl", "alkenyl", "alkynyl", "aryl", "heteroaryl", "heterocycle" refers also to the corresponding "alkylene", "cycloalkylene", "alkenylene", "alkynylene", "arylene", "heteroarylene", "heterocyclene" which are formed by the removal of two hydrogen atoms.
As used herein, the term "patient" refers to a warm-blooded animal such as a mammal, preferably a human or a human child, which is afflicted with, or has the potential to be afflicted with one or more diseases and conditions described herein.
As used herein, a "therapeutically effective amount" refers to an amount of a compound of the present invention which is effective in reducing, eliminating, treating or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.
As used herein, the term "pharmaceutically acceptable" refers to those compounds, materials, compositions, or dosage forms which are, within the scope of sound medical judgment, suitable for contact with the tissues of human beings and animals without excessive toxicity, irritation, allergic response, or other problem complications commensurate with a reasonable benefit/risk ratio.
As used herein, "pharmaceutically acceptable salts" refer to derivatives of the disclosed compounds wherein the parent compound is modified by making acid or base salts thereof. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, tartaric, citric, methanesulfonic, benzenesulfonic, glucoronic, glutamic, benzoic, salicylic, toluenesulfonic, oxalic, fumaric, maleic, and the like. Further addition salts include ammonium salts such as tromethamine, meglumine, epolamine, etc., metal salts such as sodium, potassium, calcium, zinc or magnesium.
The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418.

The compounds of the general formula (I) having geometrical and stereoisomers are also a part of the invention.

According to a further object, the present invention is also concerned with the process of preparation of the compounds of formula (I).
The compounds and process of the present invention may be prepared in a number of ways well known to those skilled in the art. The compounds can be synthesized, for example, by application or adaptation of the methods described below, or variations thereon as appreciated by the skilled artisan. The appropriate modifications and substitutions will be readily apparent and well known or readily obtainable from the scientific literature to those skilled in the art.
In particular, such methods can be found in R.C. Larock, Comprehensive Organic Transformations, VCH publishers, 1989.

It will be appreciated that the compounds of the present invention may contain one or more asymmetrically substituted carbon atoms, and may be isolated in optically active or racemic forms. Thus, all chiral, diastereomeric, racemic forms and all geometric isomeric forms of a structure are intended, unless the specific stereochemistry or isomeric form is specifically indicated. It is well known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.
Compounds of the present invention may be prepared by a variety of synthetic routes. The reagents and starting materials are commercially available, or readily synthesized by well-known techniques by one of ordinary skill in the arts. All substituents, unless otherwise indicated, are as previously defined.

In the reactions described hereinafter it may be necessary to protect reactive functional groups, for example hydroxy, amino, imino, thio or carboxy groups, where these are desired in the final product, to avoid their unwanted participation in the reactions. Conventional protecting groups may be used in accordance with standard practice, for examples see T.W. Greene and P. G. M. Wuts in Protective Groups in Organic Chemistry, John Wiley and Sons, 1991; J. F. W. McOmie in Protective Groups in Organic Chemistry, Plenum Press, 1973.
Some reactions may be carried out in the presence of a base. There is no particular restriction on the nature of the base to be used in this reaction, and any base conventionally used in reactions of this type may equally be used here, provided that it has no adverse effect on other parts of the molecule. Examples of suitable bases include: sodium hydroxide, potassium carbonate, triethylamine, alkali metal hydrides, such as sodium hydride and potassium hydride; alkyllithium compounds, such as methyllithium and butyllithium; and alkali metal alkoxides, such as sodium methoxide and sodium ethoxide.

Usually, reactions are carried out in a suitable solvent. A variety of solvents may be used, provided that it has no adverse effect on the reaction or on the reagents involved. Examples of suitable solvents include: hydrocarbons, which may be aromatic, aliphatic or cycloaliphatic hydrocarbons, such as hexane, cyclohexane, benzene, toluene and xylene; amides, such as dimethylformamide; alcohols such as ethanol and methanol and ethers, such as diethyl ether and tetrahydrofuran.
The reactions can take place over a wide range of temperatures. In general, we find it convenient to carry out the reaction at a temperature of from 0°C to 150°C (more preferably from about room temperature to 100°C). The time required for the reaction may also vary widely, depending on many factors, notably the reaction temperature and the nature of the reagents. However, provided that the reaction is effected under the preferred conditions outlined above, a period of from 3 hours to 20 hours will usually suffice.

The compound thus prepared may be recovered from the reaction mixture by conventional means. For example, the compounds may be recovered by distilling off the solvent from the reaction mixture or, if necessary after distilling off the solvent from the reaction mixture, pouring the residue into water followed by extraction with a water-immiscible organic solvent and distilling off the solvent from the extract. Additionally, the product can, if desired, be further purified by various well known techniques, such as recrystallization, reprecipitation or the various chromatography techniques, notably column chromatography or preparative thin layer chromatography.

According to an object of the invention, the process of preparation of a compound of formula (I) of the invention comprises the step of reacting a compound of formula (III): 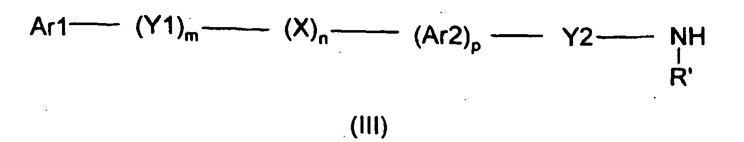 with a compound of formula (IV):

O=(Y3')_{q}-Z (CO₂R") (COR"') (IV)

wherein Ar1, Y1, X, Ar2, Y2, R', Z, R", R"', m, n, p, q are defined as in formula (I), provided that when q=0, the keto function is attached to a carbon atom of Z, and when q=1, Y3' is such that - CH2-Y3'- corresponds to Y3 as defined in formula (I).
Generally, this coupling reaction is performed out under reductive amination conditions. Preferably, this reaction is carried out by mixing compound (III), optionally in a suitable solvent such as dichloromethane, an ester, preferably ethyl acetate or isopropylacetate, an alcohol preferably ethanol and methanol, or an ether preferably diethyl oxide and tetrahydrofuran, with compound (IV), optionally in a suitable solvent such as dichloromethane, an ester preferably ethyl acetate and isopropylacetate, an alcohol preferably ethanol and methanol, or an ether preferably diethyl oxide and tetrahydrofuran, preferably at room temperature. Then, a suitable reductive agent, such as sodium cyanoborohydride or similar agents such as sodium triacetoxyborohydride, sodium borohydride, tetramethylammonium triacetoxyborohydride, borane-pyridine, dimethylamineborane, triethylamine-borane, tetrahydrofuran-borane, dimethylsulfane-borane, dimethylaniline-borane, diethylaniline-borane is added with eventually a Bronsted or Lewis acid such as hydrochloric acid, acetic acid, trifluoroacetic acid, titanium tetrachloride, zinc chloride and zinc trifluoroacetate, preferably at temperature comprised between 0°C and room temperature, more preferably between 5 and 10°C, and the reaction mixture is then allowed to react for a sufficient time to obtain a satisfactory rate.
If necessary, the coupling reaction is followed by further modifying Ar1, Y1, X, Ar2, Y2, R', Z, R", R'" of the compound of formula (I) obtained so as to obtain the desired compound of formula (I). For example, if in the obtained compound of formula (I), R" represents an alkyl group or R"' represents a -Oalkyl group, the desired compound of formula (I) in which R" represents a hydrogen atom or R"' represents a -OH group can be obtained by saponifying the obtained compound of formula (I). This reaction can be conducted once or twice depending on the number of ester groups to be saponified in the obtained compound of formula (I). This reaction is well known by the skilled person and can be generally conducted under usual conditions, such as in the presence of a base (such as sodium hydroxide or any other suitable base), in a solvent such as an alcohol, including ethanol.

Further, the process of the invention may also comprise the additional step of isolating the compound of formula (I). This can be done by the skilled person by any of the known conventional means, such as the recovery methods described above.

The compound of formula (III) may be obtained by reacting a compound of formula (V):

Ar1- (Y1)ₘ- (X)ₙ- (Ar2)ₚ- Y2-OSO2Alkyl (V)

with ammonia, such as methanolic ammonia, optionally followed by alkylation of the amino group with an aldehyde under reductive amination conditions such as those described in Organic Reactions, Vol 59, J. Wiley & Sons, 2002 or by alkylating a compound of formula (V) with an amine R'NH₂ so as to form the-NHR' group desired.

The compound of formula (V) is in turn obtained by reacting a compound of formula (Vl):

Ar1-(Y1)ₘ-(X)ₙ-(Ar2)ₚ-Y2-OH (VI)

with a suitable alkyl sulfonyl chloride derivative, preferably under basic conditions, preferably in the presence of an organic base such as triethylamine or similar.
The compound of formula (VI) may obtained from the corresponding aldehyde derivative (VII):

Ar1-(Y1)ₘ-(X)ₙ-(Ar2)ₚ-Y2=O (VII)

by reducing the aldehyde into the desired alcohol function.
The compound of formula (VII) is commercially available or may be obtained by applying or adapting any known methods or those described in the examples to obtain the desired compound of formula (VII) from available starting products.

The compound of formula (IV) may be obtained from a compound of formula (VIII):

HO-(Y3')_{q}-Z(CO₂R")(COR"') (VIII)

under oxidizing conditions with a chromium oxide derivative such as Jones reagent and pyridinium chlorochromate, activated dimethylsulfoxide reagents such as Swern and Moffatt, sodium or calcium hypochlorite, sodium periodate with a ruthenium salt, sodium permanganate, potassium permanganate, in an inert solvent such as dichloromethane, an alkane preferably cyclohexane, hexane and heptane, an ester preferably ethyl acetate and isopropyl acetate, or a mixture of these.
The compound of formula (VIII) is commercially available or may be obtained by applying or adapting any known methods or those described in the examples to obtain the desired compound of formula (VIII) from available starting products.

Of course, the compounds of the present invention can also be obtained in various procedures involving the components of the general formula A, B and C as follows.

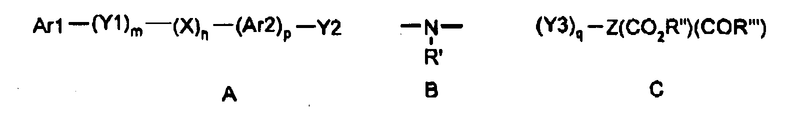

The amine component may be made as part of component A to form AB or with component C to form BC, by means of suitable synthetic strategy, and the steps may be performed according to the methods known in the art, e.g. like nucleophilic displacement or reductive amination or use of precursors like nitro functionality, etc. The synthesis may also be carried out in one pot as a multicomponent reaction.

According to a further object, the present invention is also concerned with pharmaceutical compositions comprising a compound of formula (I) together with a pharmaceutically acceptable excipient or carrier.

According to a still further object, the present invention is also concerned with the use of a compound of formula (I) 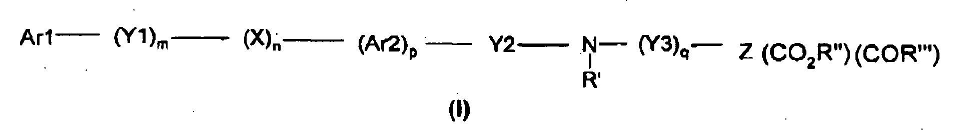 wherein
■ Ar1 represents an Aryl group optionally substituted by 1 up to 5 R group(s),
   wherein, each R, identical or different, is selected from the group consisting In Halogen atom, -Alkyl, -OAlkyl, -OH, -COOR7, -CONR7R8, -Alkyl-Hal, -OAlkyl-Hal, NO₂, -CN, -NR7R8, -AlkylAryl, -Aryl, -S(O)ₗR7, -Alkenyl, -Si(Alkyl)₃;
   wherein R7 and R8, identical or different, are chosen from the group consisting in H ; Alkyl optionally substituted by a Halogen atom, polyfluoroalkyl, Aryl; Heteroaryl; or R7 and R8 form together with the N atom to which they are attached a Heterocycle;
■ l is O, 1 or 2;
■ Y1 represents an -Alkyl- chain, optionally substituted by one or more R as defined above, and optionally comprising one or more insaturation(s) and/or heteroatom(s) and/or a residue chosen from the group consisting in -Alken-, -Alkyn-, -CH=N-, -N=CH-, -CH=N-O-, -O-N=CH-, -C(=O)-, -C(=O)(OR7)-, -C(=O)(NR7)-, -N=N-, -S(O)ₗ-;
■ m is 0 or 1;
■ X represents a heteroatom;
■ n is 0 or 1;
■ -Ar2- represents an -Aryl- group optionally substituted by one or more R group as defined above or wherein 2 R may form together with the atoms to which they are attached a fused cyclic, aryl or heteroaryl ring;
■ p is 0 or 1;
■ -Y2- represents an -Alkyl- chain, optionally substituted by one or more R as defined above, and optionally comprising one or more insaturation(s) or heteroatom(s) and/or a residue chosen from the group consisting in -Alken-, -Alkyn., -CH=N-, -N=CH-, -CH=N-O-, -O-N=CH-, -C(=O)-, -C(=O)O-, -C(=O)(NR7), -N=N-, -S(O)_{I}- ;
-R' represents a hydrogen atom, or an -Alkyl chain, optionally substituted by one or more R as defined above, and optionally comprising one or more insaturation(s) or heteroatom(s) and/or a residue chosen from the group consisting in -Alken-, -Alkyn-, -CH=N-, -N=CH-, -CH=N-O-, -O-N=CH, -C(=O)-, -C(=O)O-, -C(=O)(NR7)-, -N=N-, -S(O)_{I}-;
■ -Y3- represents an -Alkyl- chain, optionally substituted by one or more R as defined above, and optionally comprising one or more insaturation(s) or heteroatom(s) and/or a residue chosen from the group consisting in -Alken-, -Alkyn-, -CH=N-, -N=CH-, -CH=N-O-, -O-N=CH-, -C(=O)-, -C(=O)O-, -C(=O)(NR7)-, -N=N-, -S(O)ₗ-;
■ q is 0 or 1;
■ Z represents a -Cycloalkyl< group or an -Heterocyclic<, -Aryl<, -Hetetoaryl< group or a -CH< group or -C(alkyl)< group, or R' and Z form together with the N atom to which they are attached a Heterocyclic or Heteroaryl group;
   wherein (CO₂R") and (COR"') can be attached to the same atom or two adjacent atoms ;
■ -R" represents a hydrogen atom or an Alkyl chain, optionally substituted by one or more R as defined above, and optionally comprising one or more insaturation(s) ;
■ -R"' represents -OH, -OAlkyl, H, -NR7OR8, -NR7R8, natural or synthetic amino acids, wherein R7 and R8 are defined as above;
or R" and R"' form together a ring with the atom(s) to which they are attached to form a cyclic intramolecular bis carbonyl group, including Meldrum acid derivatives;
as well as their enantiomers, diastereoisomers, mixtures thereof, free forms and pharmaceutically acceptable salts, hydrates, solvates,
for the preparation of a medicament for the treatment and/or prevention of tissue graft and/or transplant rejection.

According to a still further object, the present invention is also concerned with the use of a compound of formula (I) for the preparation of a medicament for decreasing circulating lymphocytes in blood in a human patient in the need thereof.

According to a preferred aspect, such medicament is suitable as immunosuppressive agent. More preferably, such medicament is particularly suitable for the treatment and/or prevention of inflammation. Such medicament is particularly useful for treating and/or preventing autoimmune and chronic inflammatory diseases including multiple sclerosis, lupus erythematosus, rheumatoid arthritis, inflammatory bowel disease, psoriasis, asthma, myocarditis, atopic dermatitis, lupus erythematosus, type-1 diabetes, atherosclerosis, glomerulonephritis, lymphocytic leukemias, lymphomas, multiorgan failure, sepsis, pneumonia, etc.

According to a still further object, the present invention is also concerned with the use of a compound of formula (I) for the preparation of a medicament acting selectively as agonist of human S1 P1 receptors to be administered to a patient in the need thereof. Preferably, such medicament also acts as an agonist of human S1P2 receptors, and more preferably is substantially inactive at S1P3 receptors.

According to a still further object, the present invention also concerns the methods of treatment comprising administering an effective amount of a compound of the invention for treating and/or preventing the above conditions or disorders.
The present invention also provides methods for inhibiting selectively S1P1 receptors comprising administering an effective amount of a compound of the invention to a patient in the need thereof. Preferably, such methods are also suitable for inhibiting S1 P2 receptors, and more preferably without any substantial inhibition at S1 P3 receptors.
The identification of those subjects who are in need of treatment of herein-described diseases and conditions is well within the ability and knowledge of one skilled in the art. A clinician skilled in the art can readily identify, by the use of clinical tests, physical examination and medical/family history, those subjects who are in need of such treatment.
A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.
The amount of a compound of formula (I), which is required to achieve the desired biological effect, will vary depending upon a number of factors, including the dosage of the drug to be administered, the chemical characteristics (e.g. hydrophobicity) of the compounds employed, the potency of the compounds, the type of disease, the diseased state of the patient, and the route of administration.
In general terms, the compounds of this invention may be provided in an aqueous physiological buffer solution containing 0.1 to 10% w/v compound for parenteral administration. Typical dose ranges are from 1 µg/kg to 0.1 g/kg of body weight per day; a preferred dose range is from 0.01 mg/kg to 10 mg/kg of body weight per day. A preferred daily dose for adult humans includes 5, 50, 100 and 200 mg, and an equivalent dose in a human child. The preferred dosage of drug to be administered is likely to depend on such variables as the type and extent of progression of the disease or disorder, the overall health status of the particular patient, the relative biological efficacy of the compound selected, and formulation of the compound excipient, and its route of administration.
The compounds of the present invention are capable of being administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition, as described hereinafter. As such, typical daily dose ranges are from 0.01 to 10 mg/kg of body weight. By way of general guidance, unit doses for humans range from 0.1 mg to 1000 mg per day. Preferably the unit dose range is from 1 to 500 mg administered one to four times a day, and even more preferably from 10 mg to 300 mg, two times a day. Compounds provided herein can be formulated into pharmaceutical compositions by admixture with one or more pharmaceutically acceptable excipients. Such compositions may be prepared for use in oral administration, particularly in the form of tablets or capsules; or parenteral administration, particularly in the form of liquid solutions, suspensions or emulsions; or intranasally, particularly in the form of powders, nasal drops, or aerosols; or dermally, for example, topically or via transdermal patches.

The compositions may conveniently be administered in unit dosage form and may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents and/or adjuvant materials can be included as part of the composition. Oral compositions will generally include an inert diluent carrier or an edible carrier.

The tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bi-modal.

Preferred formulations include pharmaceutical compositions in which a compound of the present invention is formulated for oral or parenteral administration, or more preferably those in which a compound of the present invention is formulated as a tablet. Preferred tablets contain lactose, cornstarch, magnesium silicate, croscarmellose sodium, povidone, magnesium stearate, or talc in any combination. It is also an aspect of the present disclosure that a compound of the present invention may be incorporated into a food product or a liquid.
Liquid preparations for administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, vegetable oils such as olive oil, and organic esters such as ethyl oleate. Aqueous carriers include mixtures of alcohols and water, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like. Other potentially useful parenteral delivery systems for these active compounds include ethylene-vinyl acetate copolymer particles, osmotic pumps, implantable infusion systems, and liposomes.
Alternative modes of administration include formulations for inhalation, which include such means as dry powder, aerosol, or drops. They may be aqueous solutions containing, for example, polyoxyethylene-9-lauryl ether, glycocholate and deoxycholate, or oily solutions for administration in the form of nasal drops, or as a gel to be applied intranasally. Formulations for buccal administration include, for example, lozenges or pastilles and may also include a flavored base, such as sucrose or acacia, and other excipients such as glycocholate. Formulations suitable for rectal administration are preferably presented as unit-dose suppositories, with a solid based carrier, such as cocoa butter, and may include a salicylate. Formulations for topical application to the skin preferably take the form of an ointment, cream, lotion, paste, gel, spray, aerosol, or oil. Carriers which can be used include petroleum jelly, lanolin, polyethylene glycols, alcohols, or their combinations. Formulations suitable for transdermal administration can be presented as discrete patches and can be lipophilic emulsions or buffered, aqueous solutions, dissolved and/or dispersed in a polymer or an adhesive.

The invention is further illustrated but not restricted by the description in the following examples.

| Ex. | Compound | | | | | | MS (ESI⁺) |
|---|---|---|---|---|---|---|---|
| | 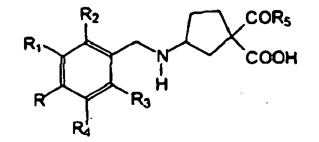 | | | | | | |

| | R | R₁ | R₂ | R₃ | R₄ | R₅ | |
|---|---|---|---|---|---|---|---|
| 1 | H₁₇C₈ | H | H | H | H | OH | 376.5 |
| 2 | H₁₇C₈ | H | H | H | H | OEt | - |
| 3 | H₁₉C₉ | H | H | H | H | OH | 390.4 |
| 4 | H₁₇C₈O | H | H | H | H | OH | 392.5 |
| 5 | H₁₇C₈O | OMe | H | H | H | OH | - |
| 6 | H₉C₄O | OMe | H | H | H | OH | 366.4 |
| 7 | H₁₃C₆O | OMe | H | H | H | OH | 394.3 |
| 8 | H₁₇C₈O | Br | H | H | OMe | OH | 502.5 & 500.4 |
| 9 | H₁₇C₈O | OMe | H | H | Me | OH | 436.5 |
| 10 | H₁₇C₈O | CI | H | H | OMe | OH | 457.4 & 455.5 |
| 11 | H₁₉C₉O | H | H | H | H | OH | 406.43 |
| 12 | H₂₁C₁₀ | H | H | H | H | OH | 404.48 |
| 13 | H₁₉C₉O | OMe | H | H | H | OH | 436.47 |
| 14 | H₁₉C₉O | CI | H | H | H | OH | 442.5 & 440.5 |

| Ex | Compound | | | | | | MS (ESI⁺) |
|---|---|---|---|---|---|---|---|
| 15 | 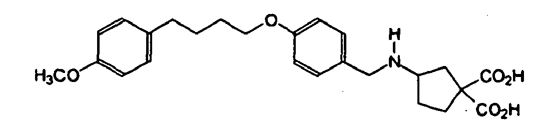 | | | | | | 442.5 |
| 16 | 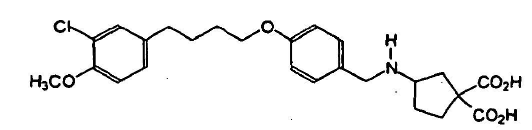 | | | | | | 478.4 & 476.4 |
| | 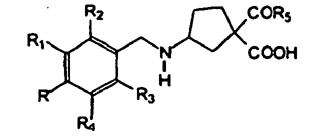 | | | | | | |

| | R | R₁ | R₂ | R₃ | R₄ | R₅ | |
|---|---|---|---|---|---|---|---|
| 17 | H₁₉C₉ | H | H | H | H | OEt | 418.3 |
| 18 | H₁₃C₆n-4-Ph | H | H | H | F | OH | 442.3 |
| 19 | H₁₉C₉ | H | H | H | H | NHCH₂ CF₃ (Isomer A) | 471.3 |
| 20 | H₁₉C₉ | H | H | H | H | NHCH₂ CF₃ (Isomer B) | 471.1 |
| 21 | H₂₁C₁₀O | H | H | H | H | OH | 420.3 |
| 22 | H₁₉C₉ | H | H | H | H | NHMe (Isomer A) | 403.4 |
| 23 | H₁₉C₉ | H | H | H | H | NHMe (Isomer B) | 403.4 |
| 24 | 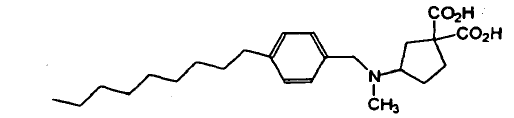 | | | | | | 404.2 |
| 25 | 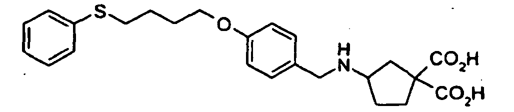 | | | | | | 444.3 |
| 26 | 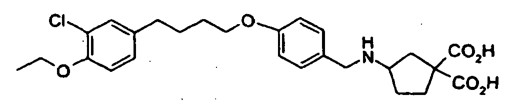 | | | | | | 492.2 & 490.2 |
| | 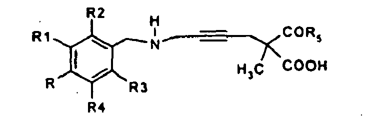 | | | | | | |
| 27 | H₁₉C₉ | H | H | H | H | OH | 402.3 |
| 28 | H₁₉C₉O | Cl | H | H | H | OH | 454.2 & 452.3 |
| 29 | H₁₇C₈ | H | H | H | H | OH | 388.3 |
| 30 | H₂₁C₁₀ | H | H | H | H | OH | 416.3 |
| 31 | H₁₃C₆n-4-Ph | H | H | H | F | OH | 454.3 |

### GENERAL EXPERIMENT PROCEDURE

### A- Preparation of 3-oxocyclopentane-1,1-dicarboxylic acid diethyl ester

### Step I

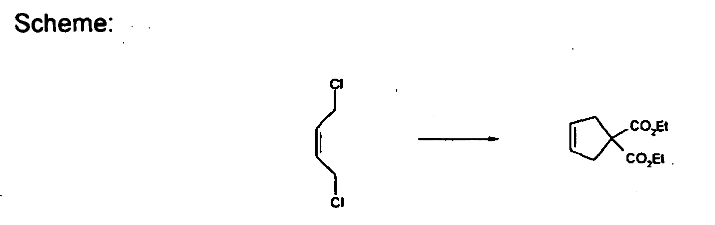

A solution of diethyl malonate (34.59 g, 0.216 mole) in THF (100 mL) is added to a solution of sodium hydride (60% w/w, 21.6 g, 0.54 mole) in THF (200 mL). The mixture is heated under reflux for 1 h, *cis*-1,4-dichlorobut-2-ene (27 g, 0.216 mole) in THF (100 mL) is added dropwise to the reaction mixture at 75°C over a period of 15 min. The reaction mixture is heated under reflux for 5 h, cooled to room temperature and quenched with water (50 mL). After removal of solvent the residue obtained is extracted with ethyl acetate (2 x 100 mL), combined organic extracts are washed with brine and dried over anhydrous sodium sulphate. The crude product obtained upon solvent removal under reduced pressure is purified by column chromatography (silica gel 230-400 mesh, ethyl acetate : hexane 10:90) to get diethyl ester of cyclopent-3-ene-1, 1-dicarboxylic acid.

### Step II

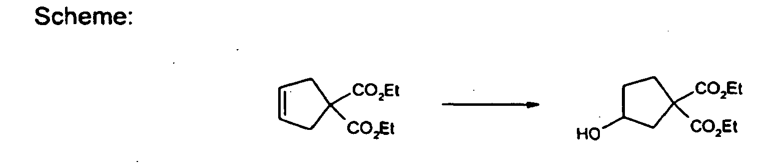

A solution of cyclopent-3-ene-1,1-dicarboxylic acid diethyl ester (15 g, 0.0706 mole) in THF (40 mL) is added slowly to mercuric acetate (24.77 g, 0.078 mole) in water-THF (110 mL, 7:4) at room temperature and stirred for 10 minutes. Perchloric acid (0.5 mL) is added to the reaction mixture (yellow ppt. disappears) continued stirring for 30 minutes. The reaction mixture is cooled to ~5°C and a solution of sodium hydroxide (4.24 g, in 32 mL water) and sodium borohydride (4.27 g, 0.112 mole) is added. The reaction mixture is stirred for 1.25 h and filtered through celite. The organic layer is separated from the filtrate and the aqueous layer is extracted with ethyl acetate (2x30 mL). Combined organic layers are washed with brine and dried over anhydrous sodium sulphate. Solvent is removed under reduced pressure and the residue is purified by column chromatography (silica gel 230-400 mesh, n-hexane:ethyl acetate 1:1) to get 3-hydroxycyclopentane-1,1-dicarboxylic acid diethyl ester.

### Step III

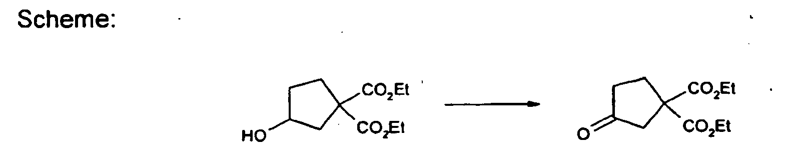

Jone's reagent (11.84 mL) is added dropwise to a solution of 3-hydroxycyclopentane-1,1-dicarboxylic acid diethyl ester (1.8 g, 0.0078 mole) in acetone (20 mL) at 10-15°C. The mixture is stirred at room temperature for 1 h, further quantity of the reagent (11.84 mL) is added and stirred for 2 more hours. The reaction is treated with isopropanol (5 mL) and concentrated under reduced pressure. Water is added to the residue and extracted with ethyl acetate (2x30 mL). Combined organic extracts are washed with brine and dried over anhydrous sodium sulphate. Removal of solvent under reduced pressure yields diethyl ester of 3-oxocyclopentane-1,1-dicarboxylic acid.

### B - Preparation of 3-bromo-5-methoxy-4-octyloxybenzylamine

### Step-I

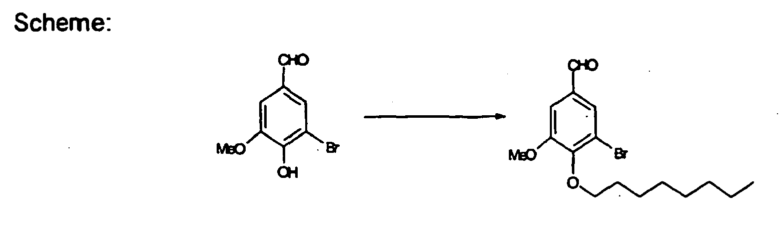

1-lodooctane (9.4 mL, 0.051 mole) is added to a solution of 5-bromovanillin (10.0 g, 0.043 mole) in DMF (50 mL) at room temperature. Potassium carbonate (9.55 g, 0.069 mole) is added and the reaction mixture is heated at 120°C for 1.5 h. Reaction mixture is concentrated under vacuum, water (50 mL) is added to the residue and extracted with ethyl acetate (3x30 mL). The combined organic layers are washed with water (1x20 mL) followed by brine solution (1x25 mL) and dried over Na₂SO₄. Removal of ethyl acetate gives light brown viscous liquid which is purified by column chromatography (silica gel 230-400 mesh, n-hexane-ethyl acetate, 92:8) to get 3-bromo-5-methoxy-4-octyloxybenzaldehyde.

### Step-II:

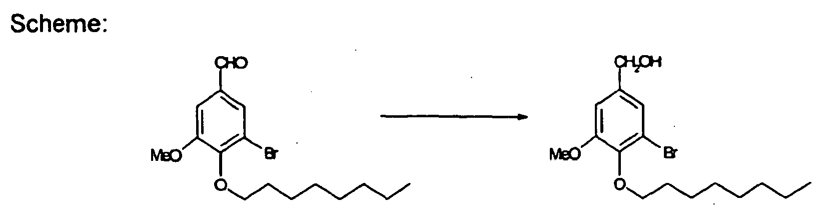

Sodium borohydride (0.27 g, 0.0073 mole) is added in portions to a stirred THF (30 mL) solution of 3-bromo-5-methoxy-4-octyloxybenzaldehyde (5 g, 0.0146 mole) at room temperature and stirred for 45 min. The reaction mixture is concentrated under vacuum, water (20 mL) is added to the residue and extracted with dichloromethane (3x25 mL). The combined organic layers are washed with water (1x15 mL) followed by brine solution (1x15 mL) and dried over anhydrous Na₂SO₄. Removal of solvent furnishes 3-bromo-5-methoxy-4-octyloxyphenylmethanol.

### Step-III

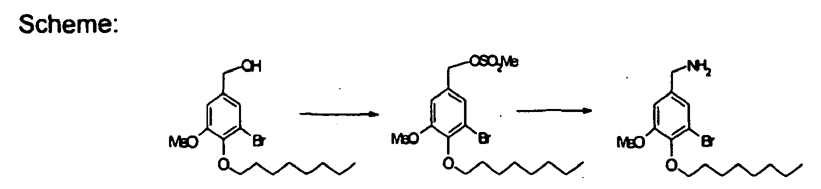

Triethylamine (8.0 mL, 0.0578 mole) is added to a dichloromethane solution (40 mL) of 3-bromo-5-methoxy-4-octyloxybenzylalcohol (4.98 g; 0.0144 mole). Reaction mixture is cooled to 0° to -5° C and methanesulphonyl chloride (3.4 mL, 0.0433 mole) is added dropwise and stirred for 10 minutes at 0°-5° C, followed by stirring at room temperature for 3.5 h. Reaction mixture is quenched with water (15 mL) under ice cold condition and aqueous layer is extracted with dichloromethane (3x25 mL). The combined dichloromethane layers are washed with water (1x20 mL) followed by brine solution (1x20 mL) and dried over anhydrous Na₂SO₄. Removal of dichloromethane furnishes crude 3-bromo-5-methoxy-4-octyloxybenzyl mesylate.

Crude 3-bromo-5-methoxy-4-octyloxybenzyl mesylate is dissolved in methanol (10 mL) and cooled to 0° to -5° C . Methanolic ammonia (~100 mL) is added to the cooled reaction mixture and stirred for 10 min, followed by stirring at room temperature for 18 h. Excess ammonia is removed and reaction mixture is concentrated under vacuum. The crude material is purified by column chromatography (silica gel 230-400 mesh, methanol : ethyl acetate; 20:80) to obtain the desired 3-bromo-5-methoxy-4-octyloxybenzylamine.

The following benzylamines were also prepared using procedure analogous to the one described above :

| Example | Compound | | | | |
|---|---|---|---|---|---|
| | 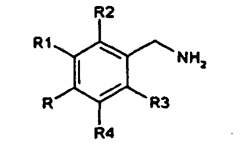 | | | | |

| | R | R₁ | R₂ | R₃ | R₄ |
|---|---|---|---|---|---|
| 4 | H₁₇C₈O | H | H | H | H |
| 5 | H₁₇C₈O | OMe | H | H | H |
| 6 | H₉C₄O | OMe | H | H | H |
| 7 | H₁₃C₆O | OMe | H | H | H |
| 9 | H₁₇C₈O | OMe | H | H | Me |
| 10 | H₁₇C₈O | Cl | H | H | OMe |
| 11 | H₁₉C₉O | H | H | H | H |
| 13 | H_{1g}C₉O | OMe | H | H | H |
| 14 | H_{1g}C₉O | Cl | H | H | H |
| 15 | 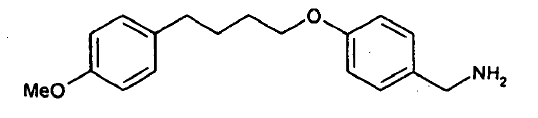 | | | | |
| 16 | 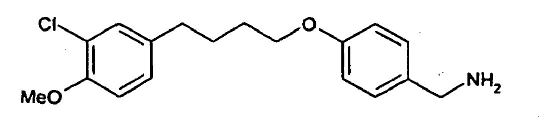 | | | | |

### C- Preparation of 4-nonylbenzylamine

### Step I

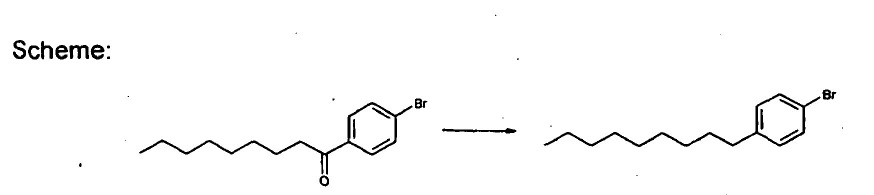

Triethylsilane (20 mL) is added dropwise to a solution of 1-(4-bromophenyl)nonan-1-one (10 g, 0.033 mole) in TFA (20 mL) stirred at room temperature. The mixture is then heated under reflux for 4.5 h. The reaction mixture is cooled and concentrated under reduced pressure. The resulting residue is purified by column chromatography (silica gel 230-400 mesh, n-hexane:ethyl acetate 95:5) to get 1-bromo-4-nonylbenzene.
Ref: J. Org. Chem. 38, 2675 (1973)

### Step II

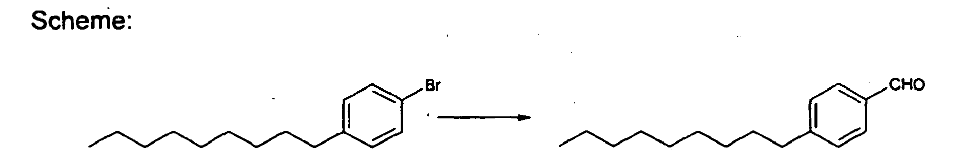

To a heterogeneous solution of magnesium (0.771 g, 0.032 mole) in THF ((10 mL) under nitrogen atmosphere are added few crystals of iodine, followed by 5 mL of 1-bromo-4-nonylbenzene (6.5 g, 0.0229 mole) in THF (40 mL) with vigorous stirring at 65 °C. After initiation, rest of the solution of 1-bromo-4-nonylbenzene is added dropwise to the reaction mixture and heated under reflux for 1 h. The reaction mixture is cooled to 0 to -5°C, a solution of THF and DMF (15 mL + 3.5 mL) is added and stirred at room temperature for 3 h. The reaction is quenched with 6N HCI (50 mL) at 0 to -5°C and extracted with ethyl acetate (2x40 mL). Combined organic layers are washed with brine (1x15 mL) and dried over Na₂SO₄. Removal of solvent gives crude viscous liquid which is purified by column chromatography (silica gel 230-400 mesh, n-hexane:ethyl acetate 95:5) to get 4-nonylbenzaldehyde.

### Step III

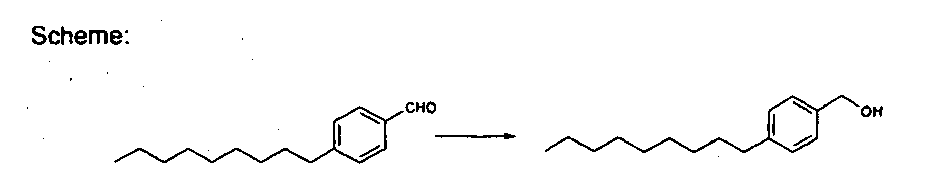

Sodium borohydride (0.847 g. 0.022 mole) is added portionwise to a solution of 4-nonylbenzaldehyde (2.6 g, 0.011 mole) in methanol (30 mL) over a period of 15 min at room temperature and further allowed to stir for 2 h. The reaction is concentrated under reduced pressure, quenched with water (10 mL) and extracted with ethyl acetate (3x30 mL). Combined organic extracts are washed with brine (1x15 mL) and dried (Na₂SO₄). Removal of solvent furnishes 4-nonylphenylmethanol.

### Step IV

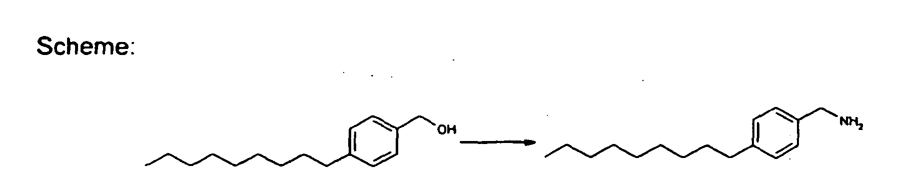

Methane sulphonyl chloride (1.9 g., 0.016 mole) is added drop wise to a solution of 4-nonylphenylmethanol (2.6 g., 0.011 mole) and triethyl amine (2.24 g., 0.022 mole) in MDC (30 ml) at 0 to 5°C. The reaction mixture is allowed to stir at room temperature for 2 hrs, quenched with water (20 ml) and extracted with MDC (2x25 ml). Combined organic extract is washed with brine (1x15 ml) and dried (Na₂SO₄). Removal of solvent furnished crude mesylated product.

To this mesylated product methanolic ammonia (50 ml) is added and allowed to stir at room temperature for overnight. Solvent is removed under vacuum and the residue is purified by column chromatography (silica gel 230-400 mesh, methanol :ethyl acetate 30:70) to get 4-nonylbenzylamine.

The following benzyl amines were also prepared using procedure analogous to the described above :

| Example | Compound | | | | |
|---|---|---|---|---|---|
| | 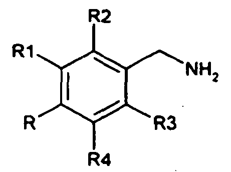 | | | | |

| | R | R₁ | R₂ | R₃ | R₄ |
|---|---|---|---|---|---|
| 1 | H₁₇C₈ | H | H | H | H |
| 12 | H₂₁C₁₀ | H | H | H | H |

### D. Preparation of 3-oxocyclopentane-1,1-dicarboxylic acid ethyl ester.

### Step I

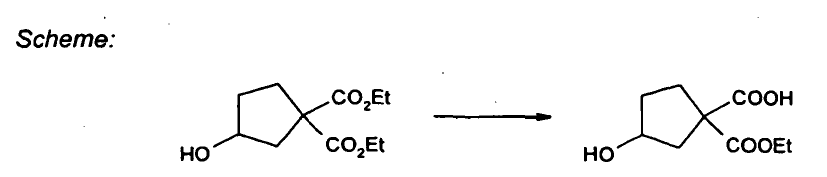

To a stirred solution of 3-hydroxycyclopentane-1,1-dicarboxylic acid diethyl ester (0.85 g, 0.0037 mole) in ethanol (7 mL), a solution of sodium hydroxide (0.162 g, 0.0040 mole) in water (3 mL) is added at room temperature. The reaction mixture is heated under reflux for 1.5 h. It is then concentrated under reduced pressure and the residue is quenched with water (10 mL). The aqueous layer after washing with ethyl acetate (1x10 mL) is acidified to pH -2 by dropwise addition of dil. HCl. It is then extracted with ethyl acetate (3x15 mL). Combined organic extracts are washed with brine and dried over sodium sulphate. Removal of solvent furnishes 3-hydroxycyclopentane-1,1-dicarboxylic acid ethyl ester.

### Step II

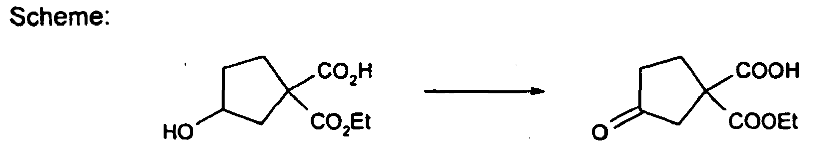

Jone's Reagent (11.88 mL) is added dropwise to a solution of 3-hydroxycyclopentane-1,1-dicarboxylic acid ethyl ester (0.8 g, 0.0040 mole) in acetone (10 mL) at 10-15°C. The mixture is stirred at room temperature for 4 h. The reaction mixture is treated with isopropanol (10 mL) and concentrated under reduced pressure. Water is added to the residue and extracted with ethyl acetate (3x10 mL). Combined organic extracts are washed with brine and dried over anhydrous sodium sulphate. Removal of solvent under reduced pressure yields 3-oxocyclopentane-1,1-dicarboxylic acid ethyl ester.

### E. Preparation of 3-oxo-1-(2,2,2-trifluoroethylcarbamoyl)cyclopentanecarboxylic acid ethyl ester.

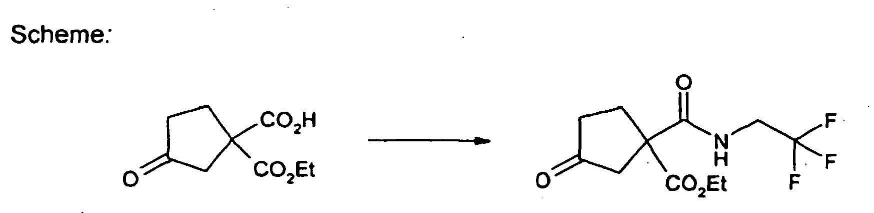

Isobutyl chloroformate (0.49 mL, 0.00378 mole) is added to a solution of 3-oxo-cyclopentane-1,1-dicarboxylic acid ethyl ester (0.75 g, 0.00375 mole) and N-methyl-morpholine (0.62 mL., 0.0056 mole) in tetrahydrofuran (10 mL) at -10 to -15°C temperature. Stir the reaction mixture at -10 to -15°C for 1 h, triethyl amine (0.78 mL, 0.0056 mole) and 2,2,2-trifluroethylamine hydrochloride (0.51 g, 0.00377 mole) are added. The reaction mixture is slowly brought to room temperature and stirred at room temperature for 1.5 h. It is then quenched with water (5 mL) and extracted with ethyl acetate (2x15 mL). Combined organic extracts are washed with saturated sodium bicarbonate solution and dried over anhydrous sodium sulphate. Removal of solvent under reduced pressure yields a viscous liquid which is purified by column chromatography (silica gel 230-400 mesh, hexane:ethyl acetate 1:1) to get 3-oxo-1-(2,2,2-trifluoroethylcarbamoyl)cyclopentanecarboxylic acid ethyl ester.

### F. Preparation of 3-[methyl-(4-nonylbenzyl)amino]-cyclopentane-1,1-dicarboxylic acid diethyl ester

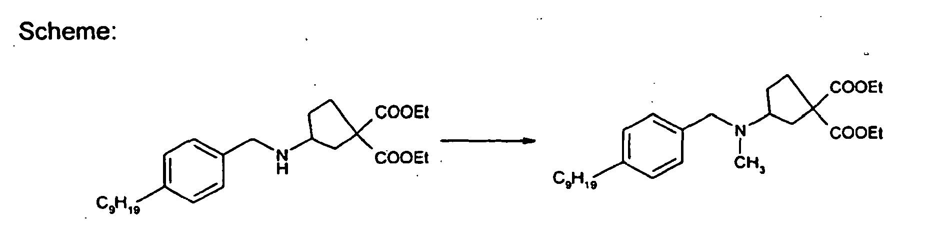

To a stirred solution of 3-(4-nonylbenzylamino)cyclopentane-1,1-dicarboxylic acid ethyl ester (0.6 g, 0.0013 mole) in acetone (15 mL) is added potassium carbonate (0.24 g, 0.0017 mole). The reaction mixture is cooled to 5-10°C and methyl iodide (0.075 mL, 0.0012 mole) is added. The reaction mixture is brought to room temperature and stirred for 4 hrs. It is then concentrated under reduced pressure, quenched with water (5 ml) and extracted with ethyl acetate (2x5 mL). Combined organic layers are washed with brine (1x5 mL). After drying over sodium sulphate removal of solvent gives a viscous liquid which is purified by column chromatography (silica gel 230-400 mesh, ethyl acetate:hexane 1:4) to get 3-[methyl-(4-nonylbenzyl)amino]cyclopentane-1,1-dicarboxylic acid diethyl ester.

### G. Preparation of 2-(4-aminobut-2-ynyl)-2-methylmalonic acid diethyl ester

### Step-I

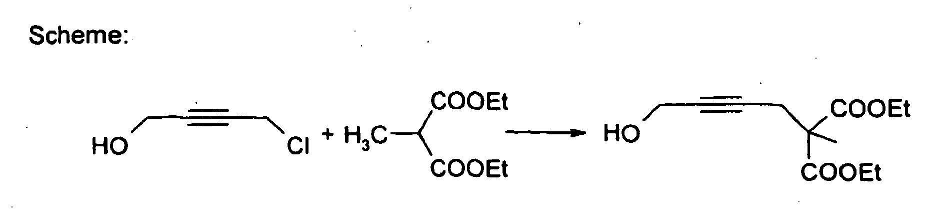

Potassium-*tert*-butoxide (6.17 g, 0.055 mole) is added to a stirred solution of diethyl methylmalonate (11.0 mL, 0.064 mole) in *N,N*-dimethylformamide (40 mL) at room temperature. The reaction mixture is heated at 90° C for one hour, cooled to room temperature and a solution of 4-chloro-but-2-yn-l-ol (4.80 g, 0.045 mole) in N,N-dimethylformamide (10 mL) is added and stirred for 2 hours at room temperature. Reaction mixture is quenched with water, concentrated under vacuum and aqueous layer is extracted with ethyl acetate (3x25 mL). The combined organic layers are washed with water (1x30 mL) followed by brine solution (1x20 mL) and dried over anhydrous Na₂SO₄. Removal of ethyl acetate gives colourless viscous liquid which is purified by column chromatography (n-hexane:ethyl acetate, 75:25) to get 2-(4-hydroxybut-2-ynyl)-2-methylmalonic acid diethyl ester.

### Step-II

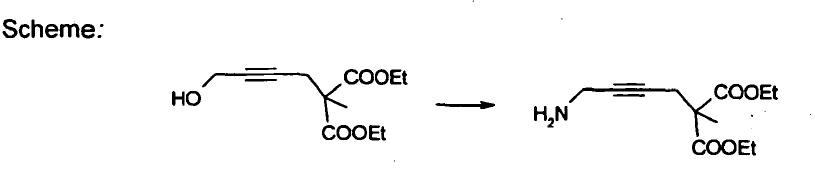

Triethylamine (3.86 mL, 0.027 mole) is added to a solution of 2-(4-hydroxybut-2-ynyl)-2-methyl malonic acid diethyl ester (4.20 g, 0.0173 mole) in dichloromethane. Methanesulphonyl chloride (1.61 mL, 0.02 mole) is added dropwise to the ice cooled (0°-5° C) reaction mixture followed by stirring at room temperature for 30 minutes. Reaction mixture is quenched with water under ice cold condition and aqueous layer is extracted with dichloromethane. The combined dichloromethane layers are washed with water (1x15 mL) followed by brine (1x15 mL) and finally dried over anhydrous sodium sulphate. Removal of dichloromethane gives mesylate derivate as a viscous liquid.
Mesylated derivative is dissolved in methanol and methanolic ammonia is added under ice cold condition. Reaction mixture is stirred for 13 h at room temperature. Excess ammonia is removed and reaction mixture is concentrated under vacuum. The crude material is purified by passing through silica gel column (ethyl acetate:methanol, 80:20) which gives 2-(4-aminobut-2-ynyl)-2-methylmalonic acid diethyl ester.

### Examples 1 and 2

### 3-(4-Octylbenzylamino)cyclopentane-1,1-dicarboxylic acid hydrochloride and 3-(4-octylbenzylamino)cyclopentane-1,1-dicarboxylic acid ethyl ester

To a stirred solution of 4-octylbenzylamine (0.53 g, 0.00242 mole) in dichloromethane (20 mL) and methanol (2 mL), a solution of 3-oxocyclopentane-1,1-dicarboxylic acid diethyl ester (0.55 g, 0.00241 mole) in dichloromethane (5 mL) is added at room temperature. The reaction mixture is cooled to 5-10°C and sodium cyanoborohydride (0.530 g, 0.0084 mole) is added. The reaction mixture is brought to room temperature and is allowed to stir at room temperature for 4 h. The reaction mixture is concentrated under reduced pressure and the residue is quenched with water (10 mL). It is extracted in ethyl acetate (2x20 mL). Combined organic layers are washed with brine (1x10 mL) and dried over sodium sulphate. Removal of solvent gives a viscous liquid which is purified by column chromatography (silica gel 230-400 mesh, mobile phase n-hexane:ethyl acetate 7:3) to get 3-(4-octylbenzylamino)cyclopentane-1,1-dicarboxylic acid diethyl ester as a colourless viscous liquid.

To a stirred solution of 3-(4-octylbenzylamino)cyclopentane-1,1-dicarboxylic acid diethyl ester (0.8 g, 0.0018 mole) in ethanol (8 mL), a solution of sodium hydroxide (0.081 g, 0.0020 mole) in water (8 mL) is added at room temperature. The reaction mixture is heated under reflux for 1 h. The reaction mixture is concentrated under reduced pressure and the residue is quenched with water (7 mL). The aqueous solution is neutralised to pH ~6 by dropwise addition of dil. HCl. It is then concentrated under reduced pressure. The residue is purified by column chromatography (silica gel 230-400 mesh, mobile phase methanol:ethyl acetate 30:70) to get 3-(4-octylbenzylamino)cyclopentane-1,1-dicarboxylic acid ethyl ester as a white solid.
To a stirred solution of 3-(4-octylbenzylamino)cyclopentane-1,1-dicarboxylic acid ethyl ester (0.6 g, 0.0014 mole) in ethanol (6 mL), a solution of sodium hydroxide (0.17 g, 0.0044 mole) in water (6 mL) is added at room temperature. The reaction mixture is heated under reflux for 5 h. The reaction mixture is concentrated under reduced pressure and the residue is quenched with water (2 mL). The aqueous solution is acidified to pH -2 by dropwise addition of dil. HCl. It is then extracted with tetrahydrofuran (2x10 mL). Combined organic layers are washed with brine (1x10 mL) and dried over anhydrous sodium sulphate. Removal of solvent under reduced pressure gives sticky solid (0.2 g.) which is washed with ether (2x10 mL) to get 3-(4-octylbenzylamino)cyclopentane-1,1-dicarboxylic acid HCl salt.

¹³C NMR : (TFA+CDCl₃; 100.61 MHz; ppm)
14.68 (q); 23.30 (t); 29.39 (t); 29.88 (t); 30.00 (t); 30.05 (t); 31.87 (t); 32.52 (t); 33.41 (t); 36.29 (t); 37.43(t); 51.60 (t); 58.36 (d); 59.56 (s); 126.98 (s); 130.16 (2d); 130.38(2d); 146.29 (s); 175.20 (s); 176.22 (s)
Following examples were prepared and characterised by their ¹³C data in the free form or as salts in a similar way.

| Ex. | Compound | | | | | | MS (ESI⁺) |
|---|---|---|---|---|---|---|---|
| | 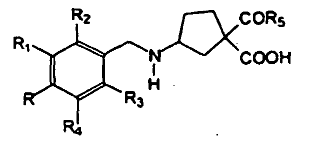 | | | | | | |

| | R | R₁ | R₂ | R₃ | R₄ | R₅ | |
|---|---|---|---|---|---|---|---|
| 1 | H₁₇C₈ | H | H | H | H | OH | 376.5 |
| 2 | H₁₇C₈ | H | H | H | H | OEt | - |
| 3 | H₁₉C₉ | H | H | H | H | OH | 390.4 |
| 4 | H₁₇C₈O | H | H | H | H | OH | 392.5 |
| 5 | H₁₇C₈O | OMe | H | H | H | OH | - |
| 6 | H₉C₄O | OMe | H | H | H | OH | 366.4 |
| 7 | H₁₃C₆O | OMe | H | H | H | OH | 394.3 |
| 8 | H₁₇C₈O | Br | H | H | OMe | OH | 502.5 & 500.4 |
| 9 | H₁₇C₈O | OMe | H | H | Me | OH | 436.5 |
| 10 | H₁₇C₈O | Cl | H | H | OMe | OH | 457.4 & 455.5 |
| 11 | H₁₉C₉O | H | H | H | H | OH | 406.43 |
| 12 | H₂₁C₁₀ | H | H | H | H | OH | 404.48 |
| 13 | H₁₉C₉O | OMe | H | H | H | OH | 436.47 |
| 14 | H₁₉C₉O | Cl | H | H | H | OH | 442.5 & 440.5 |

| Ex | Compound | | | | | | MS (ESI⁺) |
|---|---|---|---|---|---|---|---|
| 15 | 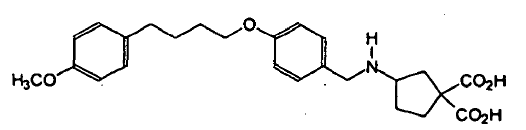 | | | | | | 442.5 |
| 16 | 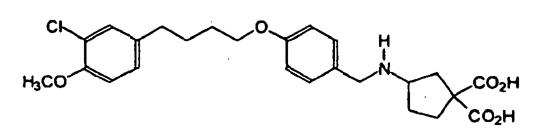 | | | | | | 478.4 & 476.4 |

### Example 3 as HCl

¹³C NMR : (CDCl₃ + TFA; 100.61 MHz; ppm)
14.66 (q); 23.32 (t); 29.48 (t); 29.97 (t); 29.98 (t); 30.10 (t); 30.18 (t); 31.86 (t); 32.55 (t); 33.59 (t); 36.29 (t); 37.40 (t); 51.77 (t); 58.54 (d); 59.82 (s); 126.78 (s); 130.19 (2d); 130.28 (2d); 146.54 (s); 175.62 (s); 176.89 (s)

### Example 4 as HCl

¹³C NMR : (CDCl₃+ TFA; 100.61 MHz; ppm)
14.66 (q); 23.28 (t); 26.57 (t); 29.28 (t); 29.75 (t); 29.86 (t); 29.99 (t); 32.45 (t); 33.15 (t); 37.44 (t); 51.28 (t); 58.17 (d); 59.51 (s); 69.06 (t); 115.95 (2d); 121.81 (s); 132.17 (2d); 160.97 (s); 174.99 (s); 175.72 (s)

### Example 5 in the free form

¹³ C NMR : (CDCl₃+TFA; 50.33MHz; ppm)
14.54 (q); 23.25 (t); 26.42 (t); 29.43 (t); 29.57 (t); 29.80 (t); 29.91 (t); 32.42 (t); 33.55 (t); 37.39 (t); 51.96 (t); 56.72 (q); 58.75 (d); 59.60 (s); 70.25 (t); 113.95 (2d); 122.18 (d); 124.08 (s); 149.92 (s); 150.53 (s); 175.67 (s); 176.87 (s).

### Example 6 as HCl

¹³ C NMR : (CDCl₃ +TFA; 100.61 MHz; ppm)
14.25 (q); 19.66 (t); 29.47 (t); 31.48 (t); 33.34 (t); 37.45 (t); 51.73 (t); 56.88 (q); 58.30 (d); 59.50 (s); 69.76 (t); 113.65 (d); 114.23 (d); 122.15 (d); 124.15 (s); 149.86 (s); 150.42 (s); 175.35 (s); 176.12 (s)

### Example 7 in the free form

¹³C NMR : (CDCl₃+TFA; 50.33 MHz; ppm)
14.45 (q); 23.12 (t); 26.08 (t); 29.45 (2t); 32.10 (t); 33.38 (t); 37.27 (t); 51.67 (t); 56.59 (q); 58.51 (d); 59.59 (s); 70.01 (t); 113.80 (2d); 122.34 (d); 123.80 (s); 150.06 (s); 150.47 (s); 175.20 (s); 176.51 (s)

### Example 8 as HCl

¹³ C NMR : (CDCl3+TFA; 100.61 MHz, ppm)
14.61 (q); 23.26 (t); 26.37 (t); 29.35 (t); 29.86 (t); 29.94 (t); 30.56 (t); 32.45 (t); 33.37 (t); 37.23 (t); 51.04 (t); 56.56 (q); 58.91 (d); 59.55 (s); 74.98 (t); 113.46 (d); 119.18 (s); 126.72 (s); 127.14 (d); 147.47 (s); 155.03 (s); 174.96 (s); 176.37 (s).

### Example 9 in the free form

¹³C NMR : (Pyridine d₅+Methanol d₄; 100.61MHz; ppm)
14.89(q); 16.93 (q); 23.75 (t); 27.26 (t); 29.31 (t); 30.43 (t); 30.55 (t); 31.37 (t); 31.60 (t); 32.95 (t); 45.31 (t); 45.83 (t); 56.68 (q); 59.48 (d); 59.91 (s); 73.65 (t); 111.56 (d); 123.53 (d); 132.97 (s); 133.22 (s); 147.57 (s); 154.37 (s); 178.22 (s); 179.13 (s)

### Example 10 in the free form

¹³ C NMR : (Pyridine d₅+Methanol d₄; 100.61 MHz; ppm)
15.09(q); 23.94 (t); 27.31 (t); 29.53 (t); 30.60 (t); 30.67 (t); 31.58 (2t); 33.13 (t); 45.48 (t); 45.63 (t); 57.28 (q); 59.90 (d); 60.01 (s); 74.68 (t); 112.74 (d); 122.29 (d); 129.66 (s); 134.71 (s); 145.78 (s); 155.81 (s); 178.20 (s): 179.65 (s)

### Example 11 as HCl

¹³C NMR : (CDCl₃ +TFA; 100.61 MHz; ppm)
14.67 (q); 23.31 (t); 26.55 (t); 29.42 (t); 29.71 (t); 29.90 (t); 30.01 (t); 30.16 (t); 32.52 (t); 33.53 (t); 37.34 (t); 51.51 (t); 58.31 (d); 59.59 (s); 69.16 (t); 116.22 (2d); 121.36 (s); 131.73 (2d); 161.23 (s); 175.31 (s); 176.61 (s)

### Example 12

¹³ C NMR : (CDCl₃+TFA; 100.61 MHz; ppm)
14.75 (q); 23.35 (t); 29.22 (t); 30.02 (t); 30.12 (t); 30.17 (t); 30.30 (t); 30.31 (t); 31.96 (t); 32.58 (t); 33.37 (t); 36.33 (t); 37.44 (t); 51.11 (t); 58.31 (d); 59.59 (s); 127.73 (s); 129.87 (2d); 130.50 (2d); 145.57 (s); 175.08 (s); 176.15 (s)

### Example 13 as HCl

¹³C NMR: (CDCl₃+TFA; 100.61 MHz; ppm)
14.65 (q); 23.29 (t); 26.48 (t); 29.25 (t); 29.56 (t); 29.89 (t); 30.01 (t); 30.13 (t); 32.49 (t); 33.13 (t); 37.40 (t); 51.60 (t); 56.86 (q); 58.30 (d); 59.50 (s); 69.98 (t); 113.52 (d); 114.36 (d); 122.47 (s); 124.11 (d); 149.85 (s); 150.30 (s); 174.98 (s); 175.70 (s)

### Example 14 as HCl

¹³C NMR : (CDCl3 + TFA); 100.61 MHz; ppm)
14.71 (q); 23.32 (t); 26.53 (t); 29.29 (t); 29.62 (t); 29.91 (t); 29.99 (t); 30.15 (t); 32.53 (t); 33.37 (t); 37.27 (t); 50.60 (t); 58.45 (d); 59.62 (s); 70.05 (t); 114.24 (d); 122.74 (s); 124.14 (s); 130.21 (d); 132.09 (d); 156.59 (s); 175.06 (s); 176.32 (s).

### Example 15 as HCl

¹³C NMR : (CDCl₃+TFA+CD₃OD; 100.61 MHz, ppm)
28.48 (t); 29.02 (t); 29.14 (t); 32.53 (t); 35.07 (t); 37.11 (t); 50.47 (t); 55.64 (q); 57.54 (d); 59.57 (s); 68.47 (t); 114.27 (2d); 115.64 (2d); 122.62 (s); 129.80 (2d); 131.73 (2d); 134.91 (s) 158.22 (s); 160.66 (s); 174.19 (s); 175.06 (s).

### Example 16 as HCl

¹³ C NMR : (CDCl₃ +TFA; 100.61 MHz, ppm)
28.38 (t); 29.25 (t); 29.78 (t); 34.24 (t); 34.97 (t); 37.58 (t); 50.05 (t); 56.79 (q); 58.46 (d); 59.28 (s); 68.38 (t); 112.75 (2d); 115.66 (d); 122.64 (s); 128.22 (2d); 130.65 (2d); 132.06 (s); 135.97 (s); 153.76 (s); 160.58 (s); 175.79(s); 177.44(s).

### Example 17

### 3-(4-nonylbenzylamino)cyclopentane-1,1-dicarboxylic acid ethyl ester

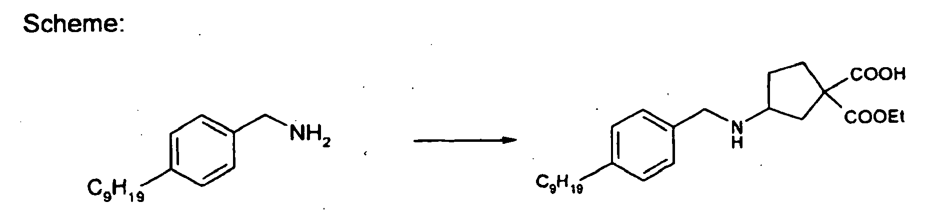

To a stirred solution of 4-nonylbenzylamine (0.2 g, 0.00086 mole) in methanol (5 mL), 3-oxocyclopentane-1,1-dicarboxylic acid ethyl ester (0.188 g, 0.00094 mole) and Triton-B (0.53 mL, 0.0013 mole) are added at room temperature. The reaction mixture is stirred for 5 min, sodium cyanoborohydride (0.08 g, 0.0013 mole) is added and it is heated under reflux for 4 h. The reaction mixture is concentrated under reduced pressure and the residue is quenched with water (5 mL). The aqueous solution is neutralised to pH ~6 by dropwise addition of dilute hydrochloric acid and extracted with tetrahydrofuran (3x10 mL). Combined organic layers are washed with brine (1x10 mL) and dried over sodium sulphate. Removal of solvent gives a viscous liquid which is purified by column chromatography (silica gel 230-400 mesh, methanol:dichloromethane1:9) to get a white solid. This is converted into the corresponding hydrochloride salt by treatment with ethanolic HCl to get 3-(4-nonylbenzylamino)cyclopentane-1,1-dicarboxylic acid ethyl ester hydrochloride.
¹³ C NMR : (CDCl₃; 100.61 MHz; ppm)
14.76 (q); 14.85 (q); 23.33 (t); 29.98 (t); -30.0 (t, probably merged); 30.05 (t); 30.15 (t); 30.21 (t); 32.01 (t); 32.54 (t); 33.69 (t); 36.34 (t); 37.57 (t); 48.75 (t); 57.56 (d); 61.50 (t); 62.62 (s); 129.59 (2d); 129.72 (s); 130.41 (2d); 144.39 (s); 173.72 (s); 179.22 (s).
The following further compounds were prepared according to the above procedures:

| Ex. | Compound | | | | | | MS (ESI⁺) |
|---|---|---|---|---|---|---|---|
| | 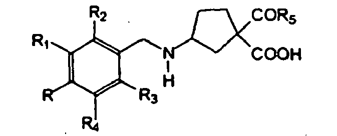 | | | | | | |

| | R | R₁ | R₂ | R₃ | R₄ | R₅ | |
|---|---|---|---|---|---|---|---|
| 17 | H₁₉C₉ | H | H | H | H | OEt | 418.3 |
| 18 | H₁₃C₆n-4-Ph | H | H | H | F | OH | 442.3 |
| 19 | H₁₉C₉ | H | H | H | H | NHCH₂CF₃ (Isomer A) | 471.3 |
| 20 | H₁₉C₉ | H | H | H | H | NHCH₂CF₃ (Isomer B) | 471.1 |
| 21 | H₂₁C₁₀O | H | H | H | H | OH | 420.3 |
| 22 | H₁₉C₉ | H | H | H | H | NHMe (Isomer A) | 403.4 |
| 23 | H₁₉C₉ | H | H | H | H | NHMe (Isomer B) | 403.4 |
| 24 | 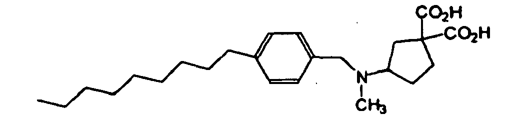 | | | | | | 404.2 |
| 25 | 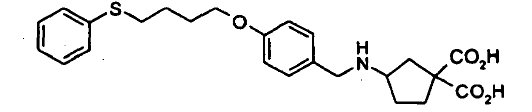 | | | | | | 444.3 |
| 26 | 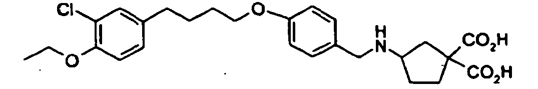 | | | | | | 492.2 & 490.2 |

### Example 18 as HCl

¹³C NMR : (CDCl₃+TFA; 100.61 MHz; ppm)
14.69 (q); 23.25 (t); 29.19 (t); 29.73 (t); 31.97 (t); 32.35 (t); 33.17 (t); 36.32 (t); 37.20 (t); 50.62 (t); 58.66 (d); 59.71 (s); 117.97 (d, J_{C-F} = 24.50 Hz); 126.32 (d); 129.30 (2d); 129.35 (2d); 130.73 (s, J_{C-F} = 7.85 Hz); 131.51 (s, J_{C-F} = 13.29 Hz); 132.29 (d); 144.06 (s); 160.35 (s, J_{C-F} = 249.91 Hz); 174.94 (s); 176.15 (s); One singlet is merged with peak having δ between 129 and 133.

### Example 19 as HCl

¹³C NMR : (TFA+CDCl₃; 100.61 MHz; ppm)
14.70 (q); 23.32 (t); 29.35 (t); 29.97 (t), 30.05 (t); 30.11 (t); 30.19 (t); 31.90 (t); 32.54 (t); 34.84 (t); 36.30 (t); 37.37 (t); 41.92 [t(q), J_{CF} =33.51 Hz]; 51.35 (t); 57.71 (d); 60.17 (s); 124.50 [s(q), J_{CF} =278.81 Hz,]; 127.07 (s); 130.08 (2d); 130.70 (2d); 146.09 (s); 172.62 (s); 175.75 (s).

### Example 20 as HCl

¹³ C NMR : (TFA+CDCl₃; 100.61 MHz; ppm)
14.65 (q); 23.30 (t); 29.96 (t); 29.99 (t), 30.09 (t); 30.12 (t); 30.17 (t); 31.87 (t); 32.53 (t); 36.29 (t); 36.87 (t); 37.67 (t); 41.82 [t(q), J_{CF} =35.19 Hz]; 51.11 (t); 59.34 (d); 59.54 (s); 124.34 [s(q), J_{CF} =278.38 Hz]; 127.32 (s): 130.15 (2d); 130.21 (2d); 146.25 (s); 175.16 (s); 175.26 (s)

### Example 21 as HCl

¹³C NMR : (TFA+CDCl₃; 100.61 MHz; ppm)
14.67 (q); 23.32 (t); 26.57 (t); 29.25 (t); 29.75 (t); 29.97 (t); 30.04 (t); 30.21 (2t); 32.55 (t); 33.22 (t); 37.24 (t); 51.23 (t); 58.16 (d); 59.69 (s); 69.05 (t): 116.05 (2d); 121.72 (s); 131.73 (2d); 161.09 (s); 174.89 (s); 176.23 (s).

### Example 22 as HCl

¹³ C NMR : (CDCl3 + TFA); 100.61MHz; ppm)
14.70 (q); 23.32 (t); 27.80 (q); 29.39 (t); 29.97 (t); 30.03 (t); 30.10 (t); 30.19 (t); 31.88 (t); 32.54 (t); 34.98 (t); 36.29 (t); 37.24 (t); 51.25 (t); 57.83 (d); 59.88 (s); 127.08 (s); 130.10 (2d); 130.48 (2d); 146.16 (s); 173.09 (s); 176.36 (s).

### Example 23 as HCl

¹³C NMR : (CDCl3 + TFA+ CD₃OD; 100.61MHz; ppm)
14.63 (q); 23.29 (t); 27.58 (q); 29.96 (t); 30.00 (t); 30.11 (t); 30.18 (t); 30.34 (t); 31.94 (t); 32.52 (t); 36.31 (t); 36.55 (t); 37.80 (t); 50.08 (t); 59.13 (d); 59.45 (s); 128.14 (s); 129.95 (2d); 130.40 (2d); 145.52 (s); 174.68 (s); 175.34 (s).

### Example 25 as HCl

¹³C NMR : (CDCl₃+TFA; 100.61 MHz, ppm)
26.25 (t); 28.78 (t); 29.21 (t); 33.29 (t); 33.88 (t); 37.26 (t); 50.73 (t); 58.11 (d); 59.62 (s); 68.02 (t); 115.71 (2d); 122.56 (s); 126.55 (d); 129.56 (2d); 129.67 (2d); 131.94 (2d); 137.11 (s); 160.56 (s); 175.04 (s); 176.19 (s).

### Example 26 as HCl

¹³C NMR : (CDCl3+TFA; 100.61 MHz, ppm)
15.33 (q); 28.30 (t); 29.17 (2t); 33.08 (t); 34.96 (t); 37.21 (t); 51.02 (t); 58.05 (d); 59.69 (s); 65.68 (t); 68.51 (t); 114.45 (d); 115.87 (d); 122.03 (s); 123.23 (s); 128.17 (2d); 130.64 (2d); 131.79 (d); 136.08 (s); 153.11 (s); 160.85 (s); 174.82 (s); 176.00 (s).

### Example 27

### 2-methyl-2-[4-(4-nonylbenzylamino)but-2-ynyl]-malonic acid hydrochloride

### Step-I

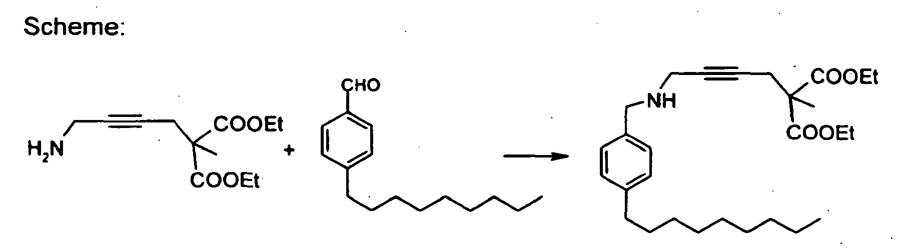

4-Nonylbenzaldehyde (1.44 g, 0.0062 mole) is added to a solution of 2-(4-aminobut-2-ynyl)-2-methylmalonic acid diethyl ester (1.50 g, 0.0062 mole) in dichloromethane at room temperature. Methanol (5 mL) is added to the reaction mixture followed by sodium cyanoborohydride (0.78 g, 0.0124 mole). After one hour stirring at room temperature, sodium cyanoborohydride (0.78 g, 0.0124 mole) is added and the mixture is stirred for 18 h at room temperature. Reaction mixture is concentrated under vacuum, water (15 mL) is added to the residue and extracted with dichloromethane (3x20 mL). The combined organic layers are washed with water (1x15 mL) followed by brine solution (1x15 mL). Removal of dichloromethane gives viscous liquid which is purified by passing through silica gel column (n-hexane:ethyl acetate, 70:30) to get 2-methyl-2-[4-(4-nonylbenzylamino)but-2-ynyl]malonic acid diethyl ester.

### Step-II

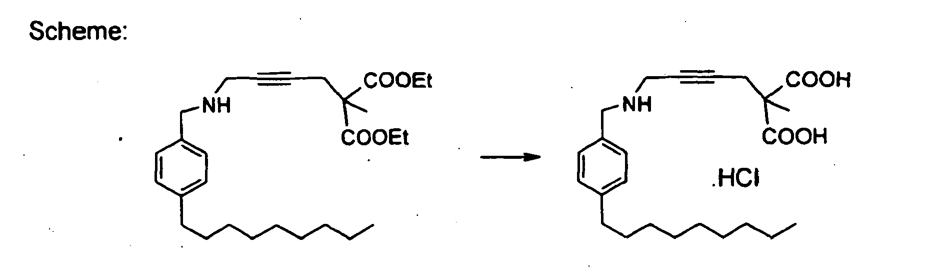

An aqueous solution (5 mL) of sodium hydroxide (0.306 g, 0.0076 mole) is added to an ethanolic solution of 2-methyl-2-[4-(4-nonylbenzylamino)but-2-ynyl]malonic acid diethyl ester (0.70 g, 0.0015 mole). The reaction mixture is then heated at 85° C for 7 h. Ethanol is removed under vacuum, water is added to the residue and acidified to pH~1 with 1 N HCl. Brine (10 mL) is added to it and extracted with tetrahydrofuran (3x15 mL). The combined organic layers are dried over sodium sulphate and concentrated under vacuum. The crude material is dissolved in minimum volume of acetone and water (5 mL) is added to it. Precipitate thus obtained is filtered and washed with water followed by 10% methanol in diethyl ether and dried under vacuum to get 2-methyl-2-[4-(4-nonylbenzylamino)-but-2-ynyl]malonic acid hydrochloride.

¹³C NMR : (CDCl₃ + TFA); 100.61 MHz; δ ppm)
14.70 (q); 20.57 (q); 23.32 (t); 26.62 (t); 29.98 (t); 30.04 (t); 30.12 (t); 30.20 (t); 31.89 (t); 32.54 (t); 36.21 (t); 36.30 (t); 50.40 (t); 53.93 (s); 72.54 (s); 86.31 (s); 126.83 (s); 130.04 (2d); 130.55 (2d); 146.02 (s); 175.11 (2s).

The following further compounds were prepared according to the above procedures:

| Ex. | Compound | | | | | | MS (ESI+) |
|---|---|---|---|---|---|---|---|
| | 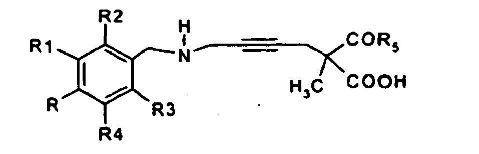 | | | | | | |
| 27 | H₁₉C₉ | H | H | H | H | OH | 402.3 |
| 28 | H₁₉C₉O | Cl | H | H | H | OH | 454.2 & 452.3 |
| 29 | H₁₇C₈ | H | H | H | H | OH | 388.3 |
| 30 | H₂₁C₁₀ | H | H | H | H | OH | 416.3 |
| 31 | H₁₃C₆n-4-Ph | H | H | H | F | OH | 454.3 |

### Example 28 as HCl

¹³C NMR : (CDCl₃ + TFA); 100.61MHz; δ ppm)
14.63 (q); 20.40 (q); 23.28 (t); 26.48 (2t); 29.57 (t); 29.87 (t); 29.94 (t); 30.11 (t); 32.49 (t); 36.32 (t); 49.85 (t); 53.88 (s); 70.07 (t); 72.18 (s); 86.47 (s); 114.29 (d); 121.93 (s); 124.30 (s); 130.37 (d); 132.29 (d); 156.75 (s); 175.15 (2s).

### Example 29 as HCl

¹³ C NMR : (CDCl₃ + TFA); 100.61 MHz; δ ppm)
14.74 (q); 21.24 (q); 23.32 (t); 26.95 (t); 29.93 (t); 30.08 (t); 30.10 (t); 31.93 (t); 32.54 (t); 36.02 (t); 36.33 (t); 49.96 (t); 53.99 (s); 72.64 (s); 86.64 (s); 127.37 (s); 129.87 (2d); 130.74 (2d); 145.48 (s); 175.64 (2s).

### Example 30 as HCl

¹³C NMR : (TFA+CDCl₃; 100.61 MHz; ppm)
14.71 (q); 20.52 (q); 23.33 (t); 26.58 (t); 29.99 (t); 30.05 (t); 30.12 (t); 30.25 (t); 30.28 (t); 31.89 (t); 32.56 (t); 36.23 (t); 36.30 (t); 50.49 (t); 53.93 (s); 72.47 (s); 86.29 (s); 126.69 (s); 130.09 (2d); 130.50 (2d); 146.15 (s); 175.13 (2s).

### Example 31 as HCl

¹³ C NMR : (TFA+CDCl₃; 100.61 MHz; ppm)
14.70 (q); 20.52 (q); 23.26 (t); 26.57 (t); 29.74 (t); 31.98 (t); 32.36 (t); 36.33 (t); 36.51 (t); 49.73 (t); 53.94 (s); 72.33 (s); 86.58 (s); 118.34 [d(d), J_{C-F} = 23.98 Hz)]; 126.64 (d); 129.27 (2d); 129.35 (2d); 130.33 [s(d), J_{C-F} = 7.56 Hz); 131.54 (s(d), J_{C-F} = 13.20 Hz)]; 132.30 (d); 132.36 (s); 143.96 (s); 160.35 [s(d), J_{C-F} = 249.79 Hz)]; 175.16 (2s).

### BIOLOGICAL TESTING

Human S1P1-5 receptor subtypes were stably expressed in HEK 293 or CHOK1 cells following transfection with corresponding plasmid constructs. Although the native cells somewhat respond to S1P, the level of expression and responsiveness of the antibiotic-resistant transfected cell lines that were selected is much higher.
When cultivated cells reached 80 % confluence, they were collected and, after lysis, cell membranes were collected by centrifugation and washed in buffer containing antiproteases.
Binding assays were performed using 5-20 µg of cell membranes suspended in 20 mM tris-HCl pH 7.4 containing 15 mM NaF and 2.5 mM deoxypyridoxine in a final volume of 250 µl. The radioligand was 0.5 nM ³H-D-erythro-dihydro-S1P incubated in the presence of bovine serum albumin for 1 h after or 2 h-preincubation. Non specific binding was defined from incubations in the presence of 5µM S1P.
GTP-γ-³⁵ S binding was performed using ~5 µg protein of cell membranes suspended in 50 mM tris-HCl pH 7.5 containing 10 mM Mg Cl₂, 100 mM NaCl and 10 µM GDP. The radioligand was 0.025 nM [³⁵S] GTP-γ-S and non specific binding determined in the presence of 10 µM non-radioactive GTP-γ-S. S1P and receptor agonists enhance the specific binding whereas inverse agonists reduce it. The maximal stimulation elicited by S1 P was taken as a reference to define full or partial agonism and calculate the intrinsic activity (i.a.) of compounds.
Typical results shown in Table 1 indicate that compounds of the invention are able to activate S1P1 (and sometimes S1P2) receptors with a potency similar to that of S1P itself (i.e. with full intrinsic activity and at nanomolar concentrations) without affecting significantly S1 P3 receptor.

**Table 1: Agonist activity of compounds and on S1P1, S1P3 and S1P2 receptors**

| | EC50 (nM) | EC50 (nM) | EC50 (nM) |
|---|---|---|---|
| | S1P1 | S1P3 | S1P2 |
| 3 | 6 (i.a=1) | >1000 (i.a~0.3) | 4 (i.a=1) |
| 12 | 13 (i.a=1) | >1000 (i.a~0.3) | 5 (i.a=1) |

## Claims

1. A compound of formula (I): 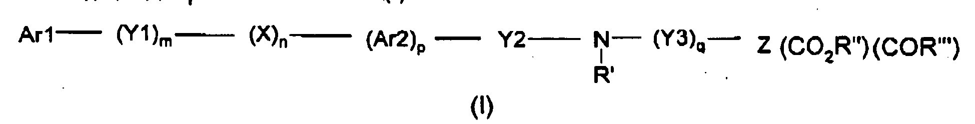 wherein
■ Ar1 represents an Aryl group optionally substituted by 1 up to 5 R group(s),
wherein, each R, identical or different, is selected from the group consisting in Halogen atom, -Alkyl, -OAlkyl, -OH, -COOR7, -CONR7R8, -Alkyl-Hal, -OAlkyl-Hal, NO₂, -CN, -NR7R8, -AlkylAryl, -Aryl, -S(O)ₗR7, -Alkenyl, -Si(Alkyl)₃;
wherein R7 and R8, identical or different, are chosen from the group consisting in H ; Alkyl optionally substituted by a Halogen atom, polyfluoroalkyl, Aryl; Heteroaryl; or R7 and R8 form together with the N atom to which they are attached a Heterocycle;
■ l is O, 1 or 2 ;
■ Y1 represents an -Alkyl- chain, optionally substituted by one or more R as defined above, and optionally comprising one or more insaturation(s) and/or heteroatom(s) and/or a residue chosen from the group consisting in -Alken-, -Alkyn-, -CH=N-, -N=CH-, -CH=N-O-, -O-N=CH-, -C(=O)-, -C(=O)(OR7)-, -C(=O)(NR7)-, -N=N-, -S(O)_{I}- ;
■ m is 0 or 1 ;
■ X represents a heteroatom;
■ n is 0 or 1;
■ -Ar2- represents an -Aryl- group optionally substituted by one or more R group as defined above or wherein 2 R may form together with the atoms to which they are attached a fused cyclic, aryl or heteroaryl ring ;
■ p is 0 or 1;
■ -Y2- represents an -Alkyl- chain, optionally substituted by one or more R as defined above, and optionally comprising one or more insaturation(s) or heteroatom(s) and/or a residue chosen from the group consisting in -Alken-, -Alkyn-, -CH=N-, -N=CH-, -CH=N-O-, -O-N=CH-, -C(=O)-, -C(=O)O-, -C(=O)(NR7), -N=N-, -S(O)_{I}- ;
-R' represents a hydrogen atom, or an -Alkyl chain, optionally substituted by one or more R as defined above, and optionally comprising one or more insaturation(s) or heteroatom(s) and/or a residue chosen from the group consisting in -Alken-, -Alkyn-, -CH=N-, -N=CH-, -CH=N-O-, -O-N=CH-, -C(=O)O-, -C(=O)(NR7)-, -N=N-, -S(O)_{I}-;
■ -Y3- represents an -Alkyl- chain, optionally substituted by one or more R as defined above, and optionally comprising one or more insaturation(s) or heteroatom(s) and/or a residue chosen from the group consisting in -Alken-, -Alkyn-, -CH=N-, -N=CH-, -CH=N-O-, -O-N=CH-, -C(=O)-, -C(=O)O-, -C(=O)(NR7)--N=N-, -S(O)_{I}-;
■ q is 0 or 1;
■ Z represents a -Cycloalkyl< group or -Aryl<, -C(alkyl)< group, or R' and Z form together with the N atom to which they are attached a Heterocyclic or Heteroaryl group;
wherein (CO₂R") and (COR"') can be attached to the same atom or two adjacent atoms ;
■ -R" represents a hydrogen atom or an Alkyl chain, optionally substituted by one or more R as defined above, and optionally comprising one or more insaturation(s) ;
-R'" represents -OH, -OAlkyl, H, -NR7OR8, -NR7R8, natural or synthetic amino acids, wherein R7 and R8 are defined as above;
or R" and R'" form together a ring with the atom(s) to which they are attached to form a cyclic intramolecular bis carbonyl group, including Meldrum acid derivatives;
as well as their enantiomers, diastereoisomers, mixtures thereof, free forms and pharmaceutically acceptable salts, hydrates, solvates
with the exception of compounds where :
- m=n=p=0, q=0 or 1, R'=H or Alkyl, Y2=-Alkyl-, Y3=-Alkyl-, Z=-C(Alkyl)< and Ar1= unsubstituted phenyl or phenyl substituted with one or more identical R where R is Halogen; and
- m=n=p=0, q= 1, R'=H, Y2=-CH₂-, Y3=-CH₂-, Z=-cyclobutyl< and Ar1= unsubstituted phenyl.

2. Compound according to claim 1 represented by the following general formula (II): 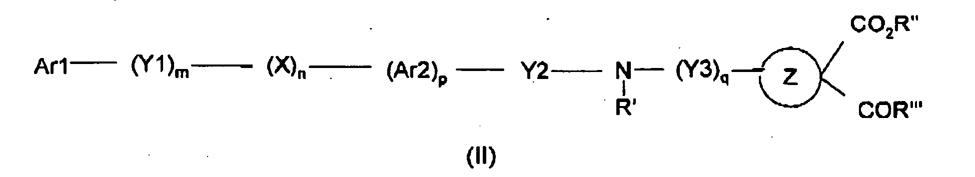 in which Ar1, Y1, X, Ar2, Y2, R, R', Y3, R", R"', m, n, p, q are defined as in formula (I) and 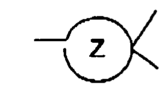 represents a -Cycloalkyl< group or an -Aryl<;
wherein (CO₂R") and (COR"') can be attached to the same atom or two adjacent atoms of the Z ring ;
as well as their enantiomers, diastereoisomers, mixtures thereof, free forms and pharmaceutically acceptable salts, hydrates, solvates.

3. Compound according to claim 1 or 2, wherein :
Ar1 represents an Aryl group optionally substituted by 1 up to 5 R group(s),
wherein each R, identical or different, is selected from the group consisting in Halogen atom, -Alkyl, -OAlkyl;
Y1 represents an -Alkyl- chain or a -S-Alkyl- chain;
m is 0 or 1;
X represents a heteroatom;
n is 0 or 1;
-Ar2- represents an -Aryl- group;
p is 0 or 1;
-Y2- represents an -Alkyl- chain;
-R' represents a hydrogen atom or an alkyl chain;
q is 0 or 1;
Y3 represents an alkynyl chain;
Z represents a -C(alkyl)< group or 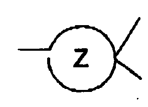 represents a -Cycloalkyl< group;
-R" represents a hydrogen atom;
-R'" represents -OH, -OAlkyl, -NR7R8;
(CO₂R") and (COR"') are attached to the same atom of the Z ring; as well as their enantiomers, diastereoisomers, mixtures thereof, free forms and pharmaceutically acceptable salts, hydrates, solvates.

4. Compound according to anyone of the preceding claims wherein Ar1 represents a Phenyl group optionally substituted by 1 up to 5 R group(s),
wherein, each R, identical or different, is selected from the group consisting in Halogen atom, -Alkyl, -OAlkyl.

5. Compound according to anyone of the preceding claims wherein Y1 represents an -Alkyl- chain or a -S-Alkyl- chain.

6. Compound according to anyone of the preceding claims wherein X represents an Oxygen atom.

7. Compound according to anyone of the preceding claims wherein -Ar2- represents an -Phenyl- group.

8. Compound according to anyone of the preceding claims wherein -Y2- represents an -Alkyl- chain.

9. Compound according to anyone of the preceding claims wherein -R' represents a hydrogen atom or an alkyl chain.

10. Compound according to anyone of the preceding claims wherein 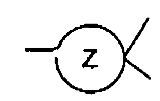 represents a -Cycloalkyl< group wherein (CO₂R") and (COR"') are attached to the same atom of the Z ring.

11. Compound according to anyone of the preceding claims wherein -R" represents a hydrogen atom.

12. Compound according to anyone of the preceding claims wherein -R' represents -OH, -OAlkyl, -NR7R8.

13. Compound according to anyone of the preceding claims selected from the group consisting in:
3-(4-octylbenzylamino)cyclopentane-1,1-dicarboxylic acid
3-(4-octylbenzylamino)cyclopentane-1,1-dicarboxylic acid ethyl ester
3-(4-nonylbenzylamino)cyclopentane-1,1-dicarboxylic acid
3-(4-octyloxybenzylamino)cyclopentane-1,1-dicarboxylic acid
3-(3-methoxy-4-octyloxybenzylamino)cyclopentane-1,1-dicarboxylic acid
3-(3-methoxy-4-butyloxybenzylamino)cyclopentane-1,1-dicarboxylic acid
3-(3-methoxy-4-hexyloxybenzylamino)cyclopentane-1,1-dicarboxylic acid
3-(3-bromo-4-octyloxy-5-methoxybenzylamino)cyclopentane-1,1-dicarboxylic acid
3-(3-methoxy-4-octyloxy-5-methylbenzylamino)cyclopentane-1,1-dicarboxylic acid
3-(3-chloro-4-octyloxy-5-methoxybenzylamino)cyclopentane-1,1-dicarboxylic acid
3-(4-nonyloxybenzylamino)cyclopentane-1,1-dicarboxylic acid
3-(4-decylbenzylamino)cyclopentane-1,1-dicarboxylic acid
3-(3-methoxy-4-nonyloxybenzylamino)cyclopentane-1,1-dicarboxylic acid
3-(3-chloro-4-nonyloxybenzylamino)cyclopentane-1,1-dicarboxylic acid
3-[4-[4-(4-methoxyphenyl)butoxy]benzylamino]cyclopentane-1,1-dicarboxylic acid
3-[4-[4-(3-chloro-4-methoxyphenyl)butoxy]benzylamino]-cyclopentane-1,1-dicarboxylic acid
3-(4-nonylbenzylamino)cyclopentane-1,1-dicarboxylic acid ethyl ester hydrochloride
3-{4-[3-fluoro-(4-hexylphenyl)benzyl]amino}cyclopentane-1,1-dicarboxylic acid hydrochloride
1-(2,2,2-trifluoroethylcarbamoyl)-3-(4-nonylbenzylamino)cyclopentanecarboxylic acid hydrochloride (isomer A)
1-(2,2,2-trifluoroethylcarbamoyl)-3-(4-nonylbenzylamino)cyclopentanecarboxylic acid hydrochloride (isomer B)
3-(4-decyloxybenzylamino)cyclopentane-1,1-dicarboxylic acid hydrochloride
1-(methylcarbamoyl)-3-(4-nonylbenzylamino)cyclopentanecarboxylic acid hydrochloride (isomer A)
1-(methylcarbamoyl)-3-(4-nonylbenzylamino)cyclopentanecarboxylic acid hydrochloride (isomer B)
3-(methyl-4-nonylbenzylamino)cyclopentane-1,1-dicarboxylic acid hydrochloride
3-[4-(4-phenylsulfanylbutoxy)benzylamino]cyclopentane-1,1-dicarboxylic acid hydrochloride
3-{4-[4-(4-ethoxy-3-chlorophenyl)butoxy]benzylamino}cyclopentane-1,1-dicarboxylic acid hydrochloride
2-methyl-2-[4-(4-nonylbenzylamino)but-2-ynyl]malonic acid hydrochloride
2-[4-(3-chloro-4-nonylbenzylamino)but-2-ynyl]-2-methylmalonic acid hydrochloride
2-methyl-2-[4-(4-octylbenzylamino)but-2-ynyl]malonic acid hydrochloride
2-[4-(4-clecylbenzylamino)but-2-ynyl]-2-methylmalonic acid hydrochloride
2-[3-fluoro-4-(4-hexylphenyl)benzylamino]but-2-ynyl]-2-methylmalonic acid hydrochloride
as well as their enantiomers, diastereoisomers, mixtures thereof, free forms and pharmaceutically acceptable salts, hydrates, solvates.

14. Compound according to anyone of the preceding claims selected from the group consisting in:
3-(4-nonylbenzylamino)cyclopentane-1,1-dicarboxylic acid
3-(4-decylbenzylamino)cyclopentane-1,1-dicarboxylic acid
as well as their enantiomers, diastereoisomers, mixtures thereof, free forms and pharmaceutically acceptable salts, hydrates, solvates.

15. Process of preparation of a compound according to anyone of the preceding claims comprising the step of reacting a compound of formula (III): 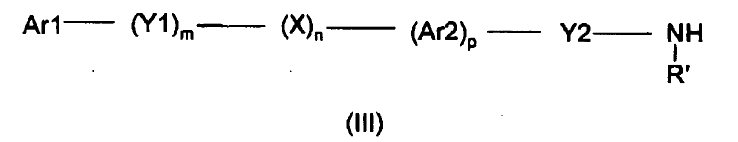 with a compound of formula (IV):
O=(Y3')_{q}- Z (CO₂R") (COR"') (IV)
wherein Ar1, Y1, X, Ar2, Y2, R', Z, R", R"', m, n, p, q are defined as in formula (I), provided that when q=0, the keto function is attached to a carbon atom of Z, and when q=1, Y3' is such that -CH2-Y3'- corresponds to Y3 as defined in formula (1).

16. Process according to claim 15 wherein said reaction is carried out under reductive amination conditions.

17. Process according to claim 15 or 16 wherein said coupling reaction is followed by further modifying Ar1, Y1, X, Ar2, Y2, R', Z, R", R'" of the compound of formula (I) obtained so as to obtain the desired compound of formula (I).

18. Process according to anyone of claims 15 to 17 which further comprises the additional step of isolating the compound of formula (I).

19. A pharmaceutical composition comprising a compound of formula (I) according to anyone of claims 1 to 14 with a pharmaceutically acceptable excipient or carrier.

20. Use of a compound of formula (I): 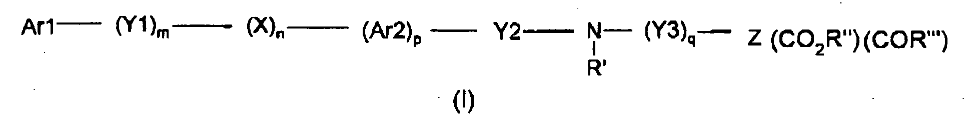 wherein
■ Ar1 represents an Aryl group optionally substituted by 1 up to 5 R group(s),
wherein, each R, identical or different, is selected from the group consisting in Halogen atom, -Alkyl, -OAlkyl, -OH, -COOR7, -CONR7R8, -Alkyl-Hal, -OAlkyl-Hal, NO₂, -CN, -NR7R8, -AlkylAryl, -Aryl, -S(O)₁R7, -Alkenyl, -Si(Alkyl)₃;
wherein R7 and R8, identical or different, are chosen from the group consisting in H ; Alkyl optionally substituted by a Halogen atom, polyfluoroalkyl, Aryl; Heteroaryl; or R7 and R8 form together with the N atom to which they are attached a Heterocycle;
■ l is O, 1 or2;
■ Y1 represents an -Alkyl- chain, optionally substituted by one or more R as defined above, and optionally comprising one or more insaturation(s) and/or heteroatom(s) and/or a residue chosen from the group consisting in -Alken-, -Alkyn-, -CH=N-, -N=CH-, -CH=N-O-, -O-N=CH-, -C(=O)-, -C(=O)(OR7)-, -C(=O)(NR7)-, -N=N-, -S(O)_{I}- ;
■ m is 0 or 1;
■ represents a heteroatom;
■ n is 0 or 1;
■ -Ar2- represents an -Aryl- group optionally substituted by one or more R group as defined above or wherein 2 R may form together with the atoms to which they are attached a fused cyclic, aryl or heteroaryl ring ;
■ p is 0 or 1;
■ -Y2- represents an -Alkyl- chain, optionally substituted by one or more R as defined above, and optionally comprising one or more insaturation(s) or heteroatom(s) and/or a residue chosen from the group consisting in -Alken-, -Alkyn-, -CH=N-, -N=CH-, -CH=N-O-, -O-N=CH-, -C(=O)-, -C(=O)O-, -C(=O)(NR7), -N=N-, -S(O)₁- ;
-R' represents a hydrogen atom, or an -Alkyl chain, optionally substituted by one or more R as defined above, and optionally comprising one or more insaturation(s) or heteroatom(s) and/or a residue chosen from the group consisting in -Alken-, -Alkyn-, -CH=N-, -N=CH-, -CH=N-O-, -O-N= CH-, -C(=O)-, -C(=O)O-, - C(=O)(NR7)-, -N=N-, -S(O)₁-;
■ -Y3- represents an -Alkyl- chain, optionally substituted by one or more R as defined above, and optionally comprising one or more insaturation(s) or heteroatom(s) and/or a residue chosen from the group consisting in -Alken-, -Alkyn-, -CH=N-, -N=CH-, -CH=N-O-, -O-N=CH-, -C(=O)-, -C(=O)O-, -C(=O)(NR7)-, -N=N-, -S(O)₁-;
■ q is 0 or 1;
■ Z represents a -Cycloalkyl< group or an -Heterocyclic<, -Aryl<, -Heteroaryl< group or a -CH< group or -C(alkyl)< group, or R' and Z form together with the N atom to which they are attached a Heterocyclic or Heteroaryl group; wherein (CO₂R") and (COR"') can be attached to the same atom or two adjacent atoms ;
■ -R" represents a hydrogen atom or an Alkyl chain, optionally substituted by one or more R as defined above, and optionally comprising one or more insaturation(s) ;
■ -R"' represents -OH, -OAlkyl, H, -NR70R8, -NR7R8, natural or synthetic amino acids, wherein R7 and R8 are defined as above;
or R" and R'" form together a ring with the atom(s) to which they are attached to form a cyclic intramolecular bis carbonyl group, including Meldrum acid derivatives;
as well as their enantiomers, diastereoisomers, mixtures thereof, free forms and pharmaceutically acceptable salts, hydrates, solvates,
for the preparation of a medicament acting as an agonist at human S1 P1 receptors selectively, for administration to a patient in the need thereof.

21. Use according to claim 20 wherein the compound of formula (I) is defined according to anyone of claims 1 to 14.

22. Use according to claim 20 or 21 wherein said medicament acts as an agonist at human S1P1and S1P2 receptors but is essentially inactive at S1P3 receptors.

23. Use according to anyone of claims 20, 21 or 22 wherein said medicament is suitable for decreasing circulating lymphocytes in blood in a patient in the need thereof.

24. Use according to anyone of claims 20 to 22 wherein said medicament is for treating and/or preventing inflammatory conditions, rheumatoid arthritis, asthma, psoriasis, myocarditis, atopic dermatitis, lymphocytic leukemias, lymphomas, multiple sclerosis, lupus erythematosus, inflammatory bowel diseases.

25. Use according to anyone of claims 20 to 22 wherein said medicament is for treating and/or preventing tissue graft rejection.

## Patentansprüche

1. Verbindung gemäß der Formel (I): 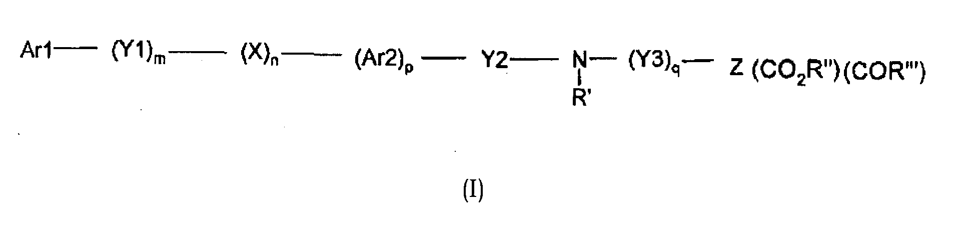 worin
**■** Ar1 eine Arylgruppe bedeutet, welche optional durch 1 bis zu 5 R-Gruppe(n) substituiert ist, wobei jedes R, gleich oder verschieden, aus der Gruppe ausgewählt ist, welche aus Halogenatom, -Alkyl, -OAlkyl, -OH, -COOR7, -CONR7R8, -Alkyl-Hal, -OAlkyl-Hal, NO₂, -CN, -NR7R8, -AlkylAryl, -Aryl, -S(O)_{I}R7, -Alkenyl, -Si(Alkyl)₃ besteht, worin R7 und R8, gleich oder verschieden, aus der Gruppe ausgewählt sind, welche aus H, Alkyl, welches optional durch ein Halogenatom substituiert ist, Polyfluoralkyl, Aryl, Heteroaryl besteht, oder R7 und R8 zusammen mit dem N-Atom, an welches diese gebunden sind, einen Heterozyklus bildet,
■ I 0, 1 oder 2 ist,
■ Y1 eine -Alkyl-Kette bedeutet, welche optional durch ein oder mehrere wie oben definierte R substituiert ist, und optional ein oder mehrere Ungesättigkeit(en) und/oder Heteroatom(e) und/oder einen Rest ausgewählt aus der Gruppe bestehend aus -Alken-, -Alkin-, -CH=N-, -N=CH-, -CH=N-O-, -O-N=CH-, -C(=O)-, -C(=O)(OR7)-, -C(=O)(NR7)-, -N=N-, -S(O)_{I}- enthält,
■ m 0 oder 1 ist,
■ X ein Heteroatom bedeutet,
■ n 0 oder 1 ist,
■ -Ar2- eine -Aryl-Gruppe optional substituiert durch eine oder mehrere wie zuvor definierte R-Gruppe(n) bedeutet oder worin 2 R zusammen mit den Atomen, an welche diese gebunden sind, einen geschlossenen Zyklus, Aryl- oder Heteroarylring bilden können,
■ p 0 oder 1 ist,
■ -Y2 eine -Alkyl-Kette bedeutet, welche optional durch ein oder mehrere wie zuvor definierte R substituiert ist, und optional ein oder mehrere Ungesättigkeit(en) oder Heteroatom(e) und/oder einen Rest ausgewählt aus der Gruppe bestehend aus -Alken-, -Alkin-, -CH=N-, -N=CH-, -CH=N-O-, -O-N=CH-, -C(=O)-, -C(=O)O-, -C(=O)(NR7)-, -N=N-, -S(O)₁- enthält, -R' ein Wasserstoffatom oder eine -Akylkette, welche optional durch ein oder mehrere wie zuvor definierte R substituiert ist, bedeutet und optional ein oder mehrere Ungesättigkeit(en) oder Heteroatom(e) und/oder einen Rest ausgewählt aus der Gruppe bestehend aus -Alken-, -Alkin-, -CH=N-, -N=CH-, -CH=N-O-, -O-N=CH-, -C(=O)O-, -C(=O)(NR7)-, -N=N-, -S(O)_{I}-enthält,
■ -Y3- eine -Alkyl-Kette bedeutet, welche optional durch ein oder mehrere wie zuvor definierte R substituiert ist, und welche optional ein oder mehrere Ungesättigtkeit(en) oder Heteroatom(e) und/oder einen Rest ausgewählt aus der Gruppe bestehend aus -Alken-, -Alkin-, -CH=N-, -N=CH-, -CH=N-O-, -O-N=CH-, -C(=O)-, -C(=O)O-, -C(=O)(NR7)-, -N=N-, -S(O)_{I}- enthält,
■ q 0 oder 1 ist,
■ Z eine -Cycloalkyl< Gruppe oder -Aryl<, -C(Alkyl)< Gruppe bedeutet oder R' und Z zusammen mit dem N-Atom, an welches diese gebunden sind, eine heterozyklische oder Heteroarylgruppe bilden,
wobei (CO₂R") und (COR"') an dasselbe Atom oder an zwei benachbarte Atome gebunden sein können,
■ -R" ein Wasserstoffatom oder eine Alkylkette, welche optional durch ein oder mehrere wie zuvor definierte R substituiert ist, bedeutet und optional ein oder mehrere Ungesättigtkeit(en) enthält,
■ -R"' -OH, -OAlkyl, H, -NR7OR8, -NR7R8, natürliche oder synthetische Aminosäuren bedeutet, worin R7 und R8 wie zuvor definiert sind,
oder R" und R"' zusammen mit dem/den Atom(en), an welche(s) diese gebunden sind, einen Ring bilden, um eine zyklische intramolekulare Biscarbonylgruppe zu bilden einschließlich Meldrum-Säurederivate,
sowie deren Enantiomere, Diastereoisomere, Mischungen hiervon, freie Formen und pharmazeutisch akzeptable Salze, Hydrate, Solvate,
mit der Ausnahme von Verbindungen, worin:
- m=n=p=0, q=0 oder 1, R'=H oder Alkyl, Y2=-Alkyl-, Y3=-Alkyl-, Z=C(Alkyl)< und Ar1=unsubstituiertes Phenyl oder Phenyl sbustituiert mit einer oder mehreren identischen R ist, worin R Halogen ist, und
- m=n=p=0, q=1, R'=H, Y=-CH₂-, Y3=-CH₂-, Z=Cyclobutyl< und Ar1=unsubstituiertes Phenyl ist.

2. Verbindung nach Anspruch 1, wiedergegeben durch die nachfolgende allgemeine Formel (II): 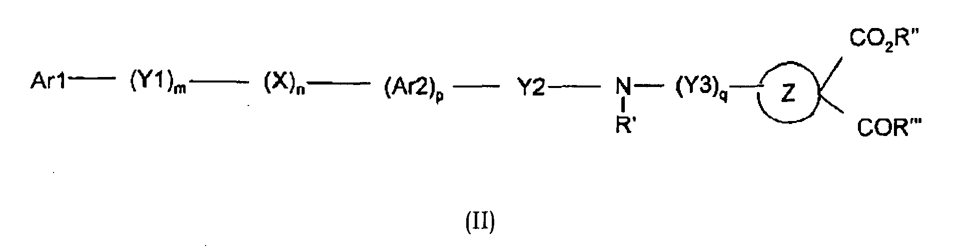 in der Ar1, Y1, X, Ar2, Y2, R, R', Y3, R", R"', m, n, p, q wie in der Formel (I) definiert sind und 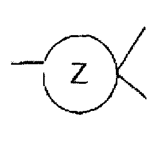 eine -Cycloalkyl< Gruppe oder eine -Aryl< bedeutet,
worin (CO₂R") und (COR"') an dasselbe Atom oder an zwei benachbarte Atome des Z-Rings gebunden sein können,
sowie deren Enantiomere, Diastereoisomere, Mischungen hiervon, freie Formen und pharmazeutisch akzeptable Salze, Hydrate, Solvate.

3. Verbindung nach Anspruch 1 oder 2, wobei:
Ar1 eine Arylgruppe bedeutet, welche optional durch 1 bis zu 5 R-Gruppe(n) substituiert ist,
worin jedes R, gleich oder verschieden, aus der Gruppe ausgewählt ist, welche aus Halogenatom, -Alkyl, -OAlkyl besteht,
Y1 eine -Alkyl-Kette oder eine -S-Alkyl-Kette bedeutet,
m 0 oder 1 ist,
X ein Heteroatom bedeutet,
n 0 oder 1 ist,
-Ar2- eine -Aryl-Gruppe bedeutet,
p 0 oder 1 ist,
-Y2- eine -Alkyl-Kette bedeutet,
-R' ein Wasserstoffatom oder eine Alkylkette bedeutet,
q 0 oder 1 ist,
Y3 eine Alkinylkette bedeutet,
Z eine -C(Alkyl)< Gruppe bedeutet oder 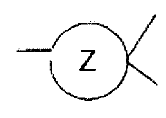 eine -Cycloalkyl< Gruppe bedeutet,
-R" ein Wasserstoffatom bedeutet,
-R"' eine -OH, -OAlkyl-, -NR7R8 bedeutet,
(CO₂R") und (COR"') an dasselbe Atom des Z-Rings gebunden sind, sowie deren Enantiomere, Diastereoisomere, Mischungen hiervon, freie Formen und pharmazeutisch akzeptable Salze, Hydrate, Solvate.

4. Verbindung nach einem der vorhergehenden Ansprüche, wobei Ar 1 eine Phenylgruppe bedeutet, welche optional durch 1 bis zu 5 R-Gruppe(n) substituiert ist, worin jedes R, gleich oder verschieden, aus der Gruppe ausgewählt ist, welche aus Halogenatom, -Alkyl, -OAlkyl besteht.

5. Verbindung nach einem der vorhergehenden Ansprüche, wobei Y 1 eine -Alkyl-Kette oder eine -S-Alkyl-Kette bedeutet.

6. Verbindung nach einem der vorhergehenden Ansprüche, wobei X ein Sauerstoffatom bedeutet.

7. Verbindung nach einem der vorhergehenden Ansprüche, wobei -Ar2- eine -Phenyl-Gruppe bedeutet.

8. Verbindung nach einem der vorhergehenden Ansprüche, wobei -Y2- eine -Alkyl-Kette bedeutet.

9. Verbindung nach einem der vorhergehenden Ansprüche, wobei -R' ein Wasserstoffatom oder eine Akylkette bedeutet.

10. Verbindung nach einem der vorhergehenden Ansprüche, wobei 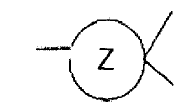 eine -Cycloalkyl< Gruppe bedeutet, wobei (CO₂R") und (COR"') an dasselbe Atom des Z-Rings gebunden sind.

11. Verbindung nach einem der vorhergehenden Ansprüche, wobei -R" ein Wasserstoffatom bedeutet.

12. Verbindung nach einem der vorhergehenden Ansprüche, wobei -R"' -OH, -OAlkyl, -NR7R8 bedeutet.

13. Verbindung nach einem der vorhergehenden Ansprüche ausgewählt aus der Gruppe bestehend aus:
3-(4-Octylbenzylamino)cyclopentan-1, 1 -dicarbonsäure
3-(4-Octylbenzylamino)cyclopentan-1,1 -dicarbonsäureethylester
3-(4-Nonylbenzylamino) cyclopentan-1,1-dicarbonsäure
3-(4-Octyloxybenzylamino)cyclopentan-1,1 -dicarbonsäure
3-(3-Methoxy-4-octyloxybenzylamino)cyclopentan-1, 1-dicarbonsäure
3-(3-Methoxy-4-butyloxybenzylamino)cyclopentan-1,1-dicarbonsäure
3-(3-Methoxy-4-hexyloxybenzylamino)cyclopentan-1, 1-dicarbonsäure
3-(3-Brom-4-octyloxy-5-methoxybenzylamino)cyclopentan-1,1-dicarbonsäure
3-(3-Methoxy-4-octyloxy-5-methylbenzylamino)cyclopentan- 1, 1 - dicarbonsäure
3-(3-Chlor-4-octyloxy-5-methoxybenzylamino) cyclopentan-1,1-dicarbonsäure
3-(4-Nonyloxybenzylamino) cyclopentan-1,1-dicarbonsäure
3-(4-Decylbenzylamino)cyclopentan- 1, 1 -dicarbonsäure
3-(3-Methoxy-4-nonyloxybenzylamino)cyclopentan-1,1-dicarbonsäure
3-(3-Chlor-4-nonyloxybenzylamino)cyclopentan- 1, 1 -dicarbonsäure
3-[4-[4-(4-Methoxyphenyl)butoxy]benzylamino]cyclopentan-1,1-dicarbonsäure
3-[4-[4-(3-Chlor-4-methoxyphenyl)butoxy]benzylamino]-cyclopentan-1, 1 -dicarbonsäure
3-(4-Nonylbenzylamino)cyclopentan-l,1-dicarbonsäureethylester-hydrochlorid
3-{4-[3-Fluor-(4-hexylphenyl)benzyl]amino}cyclopentan-1,1-dicarbonsäurehydrochlorid
1- (2, 2, 2-Trifluorethylcarbamoyl)-3-(4-nonylbenzylamino) cyclopentancarbonsäurehydrochlorid (Isomer A)
1-(2,2,2-Trifluorethylcarbamoyl)-3-(4-nonylbenzylamino)cyclopentancarbonsäurehydrochlorid (Isomer B)
3-(4-Decyloxybenzylamino)cyclopentan-1, 1-dicarbonsäurehydrochlorid
1-(Methylcarbamoyl)-3-(4-nonylbenzylamino)cyclopentancarbonsäurehydrochlorid (Isomer A)
1-(Methylcarbamoyl)-3-(4-nonylbenzylamino)cyclopentancarbonsäurehydrochlorid (Isomer B)
3-(Methyl-4-nonylbenzylamino)cyclopentan-1,1-dicarbonsäurehydrochlorid
3-[4-(4-Phenylsulfanylbutoxy)benzylamino]cyclopentan-1,1-dicarbonsäurehydrochlorid
3-{4-[4-(4-Ethoxy-3-chlorphenyl)butoxy]benzylamino}cyclopentan-1,1-dicarbonsäurehydrochlorid
2-Methyl-2-[4-(4-nonylbenzylamino)but-2-ynyl]malonsäurehydrochlorid
2-[4-(3-Chlor-4-nonylbenzylamino)but-2-ynyl]-2-methylmalonsäurehydrochlorid
2-Methyl-2-[4-(4-octylbenzylamino)but-2-ynyl]malonsäurehydrochlorid
2-[4-(4-Decylbenzylamino)but-2-ynyl]-2-methylmalonsäurehydrochlorid
2-[3-Fluor-4-(4-hexylphenyl)benzylamino]but-2-ynyl]-2-methylmalonsäurehydrochlorid
sowie deren Enantiomere, Diastereoisomere, Mischungen hiervon, freie Formen und pharmazeutisch akzeptable Salze, Hydrate, Solvate.

14. Verbindung nach einem der vorhergehenden Ansprüche, ausgewählt aus der Gruppe bestehend aus:
3-(4-Nonylbenzylamino)cyclopentan-1,1-dicarbonsäure
3-(4-Decylbenzylamino)cyclopentan-1,1-dicarbonsäure
sowie deren Enantiomere, Diastereoisomere, Mischungen hiervon, freie Formen und pharmazeutisch akzeptable Salze, Hydrate, Solvate.

15. Verfahren zur Herstellung einer Verbindung nach einem der vorhergehenden Ansprüche umfassend den Schritt des Reagierens einer Verbindung der Formel (III): 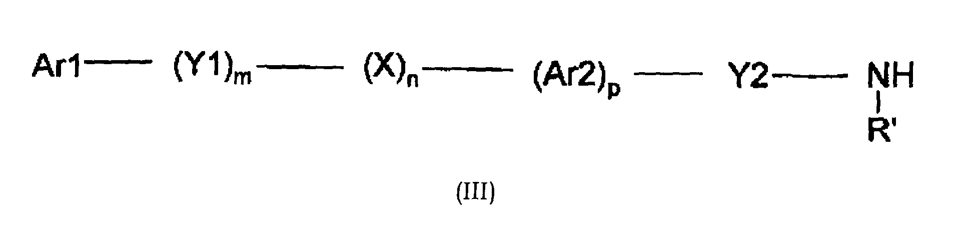 mit einer Verbindung der Formel (IV):
O=(Y3')_{q}- Z (CO₂R")(COR'") (IV)
worin Ar1, Y1, X, Ar2, Y2, R', Z, R", R"', m, n, p, q wie in der Formel (I) definiert sind, vorausgesetzt, dass, wenn q = 0 ist, die Ketofunktion an das Kohlenstoffatom von Z gebunden ist, und dass, wenn q = 1 ist, Y3' so ist, dass -CH2-Y3'- zu dem wie in der Formel (I) definierten Y3 korrespondiert.

16. Verfahren nach Anspruch 15, wobei die Reaktion unter reduktiven Aminierungsbedingungen durchgeführt wird.

17. Verfahren nach Anspruch 15 oder 16, wobei die Kupplungsreaktion vom weiteren Modifizieren von Ar1, Y1, X, Ar2, Y2, R', Z, R", R"' der erhaltenen Verbindung gemäß der Formel (I) gefolgt wird, um so die gewünschte Verbindung der Formel (I) zu erhalten.

18. Verfahren nach einem der Ansprüche 15 bis 17, welches des Weiteren den zusätzlichen Schritt des Isolierens der Verbindung gemäß der Formel (I) umfasst.

19. Pharmazeutische Zusammensetzung enthaltend eine Verbindung der Formel (I) gemäß einem der Ansprüche 1 bis 14 mit einem pharmazeutisch akzeptablen Hilfsmittel oder Träger.

20. Verwendung einer Verbindung gemäß der Formel (I): 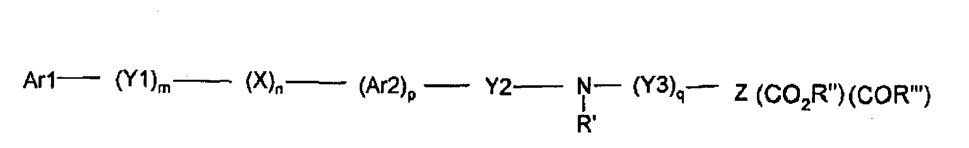 worin
■ Ar 1 eine Arylgruppe bedeutet, welche optional durch 1 bis zu 5 R-Gruppe(n) substituiert ist, wobei jedes R, gleich oder verschieden, aus der Gruppe ausgewählt ist, welche aus Halogenatom, -Alkyl, -OAlkyl, -OH, -COOR7, -CONR7R8, -Alkyl-Hal, -OAlkyl-Hal, NO₂, -CN, -NR7R8, -AlkylAryl, -Aryl, -S(O)₁R7, -Alkenyl, -Si(Alkyl)₃ besteht,
worin R7 und R8, gleich oder verschieden, aus der Gruppe ausgewählt sind, welche aus H, Alkyl, welches optional durch ein Halogenatom substituiert ist, Polyfluoralkyl, Aryl, Heteroaryl besteht, oder R7 und R8 zusammen mit dem N-Atom, an welches diese gebunden sind, einen Heterozyklus bildet,
■ I 0, 1 oder 2 ist,
■ Y1 eine -Alkyl-Kette bedeutet, welche optional durch ein oder mehrere wie oben definierte R substituiert ist, und optional ein oder mehrere Ungesättigkeit(en) und/oder Heteroatom(e) und/oder einen Rest ausgewählt aus der Gruppe bestehend aus -Alken-, -Alkin-, -CH=N-, -N=CH-, -CH=N-O-, -O-N=CH-, -C(=O)-, -C(=O)(OR7)-, -C(=O)(NR7)-, -N=N-, -S(O)_{I}- enthält,
■ m 0 oder 1 ist,
■ X ein Heteroatom bedeutet,
■ n 0 oder 1 ist,
■ -Ar2- eine -Aryl-Gruppe optional substituiert durch eine oder mehrere wie zuvor definierte R-Gruppe(n) bedeutet oder worin 2 R zusammen mit den Atomen, an welche diese gebunden sind, einen geschlossenen Zyklus, Aryl- oder Heteroarylring bilden können,
■ p 0 oder 1 ist,
■ -Y2 eine -Alkyl-Kette bedeutet, welche optional durch ein oder mehrere wie zuvor definierte R substituiert ist, und optional ein oder mehrere Ungesättigkeit(en) oder Heteroatom(e) und/oder einen Rest ausgewählt aus der Gruppe bestehend aus -Alken-, -Alkin-, -CH=N-, -N=CH-, -CH=N-O-, -O-N=CH-, -C(=O)-, -C(=O)O-, -C(=O)(NR7)-, -N=N-, -S(O)_{I}- enthält, -R' ein Wasserstoffatom oder eine -Akylkette, welche optional durch ein oder mehrere wie zuvor definierte R substituiert ist, bedeutet und optional ein oder mehrere Ungesättigkeit(en) oder Heteroatom(e) und/oder einen Rest ausgewählt aus der Gruppe bestehend aus -Alken-, -Alkin-, -CH=N-, -N=CH-, -CH=N-O-, -O-N=CH-, -C(=O)-, -C(=O)O-, -C(=O)(NR7)-, -N=N-, -S(O)₁- enthält,
■ -Y3- eine -Alkyl-Kette bedeutet, welche optional durch ein oder mehrere wie zuvor definierte R substituiert ist, und welche optional ein oder mehrere Ungesättigtkeit(en) oder Heteroatom(e) und/oder einen Rest ausgewählt aus der Gruppe bestehend aus -Alken-, -Alkin-, -CH=N-, -N=CH-, -CH=N-O-, -O-N=CH-, -C(=O)-, -C(=O)O-, -C(=O)(NR7)-, -N=N-, -S(O)_{I}- enthält,
■ q 0 oder 1 ist,
■ Z eine -Cycloalkyl< Gruppe oder eine heterozyklische<, -Aryl<, -Heteroaryl< Gruppe oder eine -CH< Gruppe oder -C(Alkyl)< Gruppe bedeutet oder R' und Z zusammen mit dem N-Atom, an welches diese gebunden sind, eine heterozyklische oder Heteroarylgruppe bilden,
wobei (CO₂R") und (COR"') an dasselbe Atom oder an zwei benachbarte Atome gebunden sein können,
■ -R" ein Wasserstoffatom oder eine Alkylkette, welche optional durch ein oder mehrere wie zuvor definierte R substituiert ist, bedeutet und optional ein oder mehrere Ungesättigtkeit(en) enthält,
■ -R'" -OH, -OAlkyl, H, -NR7OR8, -NR7R8, natürliche oder synthetische Aminosäuren bedeutet, worin R7 und R8 wie zuvor definiert sind,
oder R" und R"' zusammen mit dem/den Atom(en), an welche(s) diese gebunden sind, einen Ring bilden, um eine zyklische intramolekulare Biscarbonylgruppe zu bilden einschließlich Meldrum-Säurederivate,
sowie deren Enantiomere, Diastereoisomere, Mischungen hiervon, freie Formen und pharmazeutisch akzeptable Salze, Hydrate, Solvate,
zur Herstellung eines Arzneimittels, welches selektiv als ein Agonist bei humanen S1P1-Rezeptoren wirkt, zur Verabreichung bei einem Patienten, bei dem hierfür ein Bedarf besteht.

21. Verwendung nach Anspruch 20, wobei die Verbindung der Formel (I) wie in einem der Ansprüche 1 bis 14 definiert ist.

22. Verwendung nach Anspruch 20 oder 21, wobei das Arzneimittel als ein Agonist bei humanen S1P1- und S1P2-Rezeptoren wirkt, aber bei S1P3-Rezeptoren im Wesentlichen inaktiv ist.

23. Verwendung nach einem der Ansprüche 20, 21 oder 22, wobei das Arzneimittel zum Verringern der zirkulierenden Lymphoyzten in Blut bei einem Patienten, bei dem hierfür ein Bedarf besteht, geeignet ist.

24. Verwendung nach einem der Ansprüche 20 bis 22, wobei das Arzneimittel zum Behandeln und/oder Verhindern von Entzündungszuständen, rheumatoider Arthritis, Asthma, Psoriasis, Myokarditis, atopischer Dermatitis, lymphozytischer Leukämie, Lymphoma, multipler Sklerose, Lupus erythematosus, entzündlicher Bowel-Störung ist.

25. Verwendung nach einem der Ansprüche 20 bis 22, wobei das Arzneimittel zum Behandeln und/oder Verhindern von Gewebetransplantatabstoßung ist.

## Revendications

1. Composé de formule (I) : 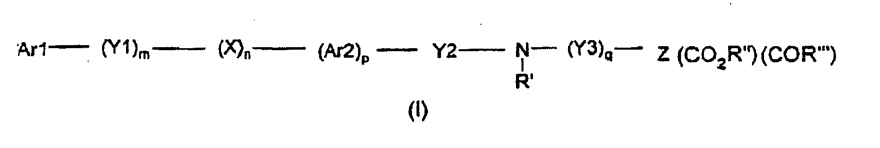 où
Ar1 représente un groupe aryle éventuellement substitué par 1 jusqu'à 5 groupes R,
où, chaque R, identique ou différent, est choisi dans le groupe constitué d'un atome d'halogène, d'un groupe -alkyle, -Oalkyle, -OH, -COOR7, -CONR7R8, -alkyl-Hal, -Oalkyl-Hal, NO₂ -CN, -NR7R8, -alkylaryle, -aryle, -S(O)₁R7, - alcényle, -Si(alkyle)₃ ;
où R7 et R8, identiques ou différents, sont choisis dans le groupe constitué de H ; un groupe alkyle éventuellement substitué par un atome d'halogène, un groupe polyfluoroalkyle, aryle ; hétéroaryle ;
ou R7 et R8 forment ensemble avec l'atome N auquel ils sont fixés un hétérocycle ;
1 vaut 0, 1 ou 2 ;
Y1 représente une chaîne -alkyl-, éventuellement substituée par un ou plusieurs R tel que défini ci-dessus, et comprenant éventuellement une ou plusieurs insaturation(s) et/ou un ou plusieurs hétéroatome(s) et/ou un résidu choisi dans le groupe constitué de -alcén-, -alcyn-, -CH=N-, -N=CH-, -CH=N-O-, -O-N=CH-, -C(=O)-, -C(=O)(OR7)-, -C(=O)(NR7)-, -N=N-, -S(O)_{I}- ;
m vaut 0 ou 1 ;
X représente un hétéroatome ;
n vaut 0 ou 1 ;
-Ar2- représente un groupe -aryl- éventuellement substitué par un ou plusieurs groupes R tel que défini ci-dessus ou bien où 2 R peuvent former ensemble avec les atomes auxquels ils sont fixés un cycle fusionné cyclique, aryle ou hétéroaryle ;
p vaut 0 ou 1 ;
-Y2- représente une chaîne -alkyl-, éventuellement substituée par un ou plusieurs R tel que défini ci-dessus, et comprenant éventuellement une ou plusieurs insaturation(s) ou un ou plusieurs hétéroatome(s) et/ou un résidu choisi dans le groupe constitué de -alcén-, -alcyn-, -CH=N-, -N=CH-, -CH=N-O-, -ON=CH-, -C(=O)-, -C(=O)O-, -C(=O)(NR7)-, -N=N-, -S(O)_{I}- ;
-R' représente un atome d'hydrogène, ou une chaîne -alkyl-, éventuellement substituée par un ou plusieurs R tel que défini ci-dessus, et comprenant éventuellement une ou plusieurs insaturation(s) ou un ou plusieurs hétéroatome(s) et/ou un résidu choisi dans le groupe constitué de -alcén-, -alcyn-, -CH=N-, -N=CH-, -CH=N-O-, -O-N=CH-, -C(=O)O-, -C(=O)(NR7)-, -N=N-, - S(O)_{I}- ;
-Y3- représente une chaîne -alkyl-, éventuellement substituée par un ou plusieurs R tel que défini ci-dessus, et comprenant éventuellement une ou plusieurs insaturation(s) ou un ou plusieurs hétéroatome(s) et/ou un résidu choisi dans le groupe constitué de -alcén-, -alcyn-, -CH=N-, -N=CH-, -CH=N-O-, -ON=CH-, -C(=O)-, -C(=O)O-, -C(=O)(NR7)-, -N=N-, -S(O)_{I}-;
q vaut 0 ou 1 ;
Z représente un groupe -cycloalkyl< ou -aryl<, un groupe -C(alkyl)<, ou R' et Z forment ensemble avec l'atome N auquel ils sont fixés un groupe hétérocyclique ou hétéroaryle ;
où (CO₂R") et (COR"') peuvent être fixés au même atome ou à deux atomes adjacents ;
-R" représente un atome d'hydrogène ou une chaîne alkyle, éventuellement substituée par un ou plusieurs R tel que défini ci-dessus, et comprenant éventuellement une ou plusieurs insaturation(s);
-R"' représente -OH, -Oalkyle, H, -NR7OR8, -NR7R8, des acides aminés naturels ou synthétiques, où R7 et R8 sont définis comme ci-dessus;
où R" et R"' forment ensemble un cycle avec le(s) atome(s) au(x)quel(s) ils sont fixés pour former un groupe bis-carbonyle intramoléculaire cyclique, y compris des dérivés de l'acide de Meldrum ;
ainsi que leurs énantiomères, diastéréo-isomères, mélanges de ceux-ci, formes libres et sels pharmaceutiquement acceptables, hydrates, solvates
à l'exception de composés où :
m = n = p = 0, q = 0 ou 1, R' = H ou alkyle, Y2 = alkyl-, Y3 = alkyl-, Z = - C(alkyl)< et Ar1 = phényle non substitué ou phényle substitué par un ou plusieurs R identiques où R représente un atome d'halogène ; et
m = n = p = 0, q = 1, R' = H, Y2 = -CH₂-, Y3 = -CH₂-, Z = -cyclobutyl< et Ar1 = phényle non substitué.

2. Composé selon la revendication 1 représenté par la formule générale (II) suivante : 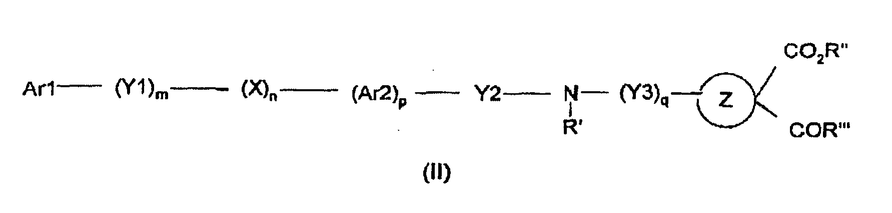 où Ar1, Y1, X, Ar2, Y2, R, R', Y3, R", R"', m, n, p, q sont définis comme dans la formule (I) et 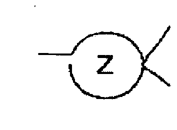 représente un groupe -cycloalkyl< ou -aryl< ;
où (CO₂R") et (COR"') peuvent être fixés au même atome ou à deux atomes adjacents du cycle Z ;
ainsi que leurs énantiomères, diastéréo-isomères, mélanges de ceux-ci, formes libres et sels pharmaceutiquement acceptables, hydrates, solvates.

3. Composé selon la revendication 1 ou 2, où :
Ar1 représente un groupe aryle éventuellement substitué par 1 jusqu'à 5 groupes R,
où chaque R, identique ou différent, est choisi dans le groupe constitué d'un atome d'halogène, d'un groupe -alkyle, -Oalkyle ;
Y1 représente une chaîne -alkyl- ou une chaîne -S-alkyl- ;
m vaut 0 ou 1 ;
X représente un hétéroatome ;
n vaut 0 ou 1 ;
-Ar2- représente un groupe -aryl- ;
p vaut 0 ou 1 ;
-Y2- représente une chaîne -alkyl- ;
-R' représente un atome d'hydrogène ou une chaîne alkyle ;
q vaut 0 ou 1 ;
Y3 représente une chaîne alcynyle ;
Z représente un groupe -C(alkyl)< ou 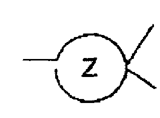 représente un groupe -cycloalkyl< ;
-R" représente un atome d'hydrogène ;
-R"' représente -OH, -Oalkyle, -NR7R8 ;
(CO₂R") et (COR"') sont fixés au même atome du cycle Z ;
ainsi que leurs énantiomères, diastéréo-isomères, mélanges de ceux-ci, formes libres et sels pharmaceutiquement acceptables, hydrates, solvates.

4. Composé selon l'une quelconque des revendications précédentes, où Ar1 représente un groupe phényle éventuellement substitué par 1 jusqu'à 5 groupes R,
où chaque R, identique ou différent, est choisi dans le groupe constitué d'un atome d'halogène, d'un groupe -alkyle, -Oalkyle.

5. Composé selon l'une quelconque des revendications précédentes, où Y1 représente une chaîne -alkyl- ou une chaîne -S-alkyl-.

6. Composé selon l'une quelconque des revendications précédentes, où X représente un atome d'oxygène.

7. Composé selon l'une quelconque des revendications précédentes, où - Ar2- représente un groupe -phényl-.

8. Composé selon l'une quelconque des revendications précédentes, où - Y2- représente une chaîne -alkyl-.

9. Composé selon l'une quelconque des revendications précédentes, où -R' représente un atome d'hydrogène ou une chaîne alkyle.

10. Composé selon l'une quelconque des revendications précédentes, où 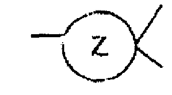 représente un groupe -cycloalkyl< où (CO₂R") et (COR"') sont fixés au même atome du cycle Z.

11. Composé selon l'une quelconque des revendications précédentes, où - R" représente un atome d'hydrogène.

12. Composé selon l'une quelconque des revendications précédentes, où - R"' représente -OH, -Oalkyle, -NR7R8.

13. Composé selon l'une quelconque des revendications précédentes, choisi dans le groupe constitué par :
l'acide 3-(4-octylbenzylamino)cyclopentane-1,1-dicarboxylique
l'ester éthylique de l'acide 3-(4-octylbenzylamino)cyclopentane-1,1-dicarboxylique
l'acide 3-(4-nonylbenzylamino)cyclopentane-1,1-dicarboxylique
l'acide 3-(4-octyloxybenzylamino)cyclopentane-1,1-dicarboxylique
l'acide 3-(3-méthoxy-4-octyloxybenzylamino)cyclopentane-1,1-dicarboxylique
l'acide 3-(3-méthoxy-4-butyloxybenzylamino)cyclopentane-1,1-dicarboxylique
l'acide 3-(3-méthoxy-4-hexyloxybenzylamino)cyclopentane-1,1-dicarboxylique
l'acide 3-(3-bromo-4-octyloxy-5-méthoxybenzylamino)cyclopentane-1,1-dicarboxylique
l'acide 3-(3-méthoxy-4-octyloxy-5-méthylbenzylamino)cyclopentane-1,1-dicarboxylique
l'acide 3-(3-chloro-4-octyloxy-5-méthoxybenzylamino)cyclopentane-1,1-dicarboxylique
l'acide 3-(4-nonyloxybenzylamino)cyclopentane-1,1-dicarboxylique
l'acide 3-(4-décyloxybenzylamino)cyclopentane-1,1-dicarboxylique
l'acide 3-(3-méthoxy-4-nonyloxybenzylamino)cyclopentane-1,1-dicarboxylique
l'acide 3-(3-chloro-4-nonyloxybenzylamino)cyclopentane-1,1-dicarboxylique
l'acide 3-[4-[4-(4-méthoxyphényl)butoxy]benzylamino]cyclopentane-1,1-dicarboxylique
l'acide 3-[4-[4-(3-chloro-4-méthoxyphényl)butoxy]benzylamino]-cyclopentane-1,1-dicarboxylique
le chlorhydrate de l'ester éthylique de l'acide 3-(4-nonylbenzylamino)cyclopentane-1,1-dicarboxylique
le chlorhydrate de l'acide 3-{4-[3-fluoro-(4-hexylphényl)benzyl]-amino} cyclopentane-1,1-dicarboxylique
le chlorhydrate de l'acide 1-(2,2,2-trifluoroéthylcarbamoyl)-3-(4-nonylbenzylamino)cyclopentane-carboxylique (isomère A)
le chlorhydrate de l'acide 1-(2,2,2-trifluoroéthylcarbamoyl)-3-(4-nonylbenzylamino)cyclopentane-carboxylique (isomère B)
le chlorhydrate de l'acide 3-(4-décyloxybenzylamino)cyclopentane-1,1-dicarboxylique
le chlorhydrate de l'acide 1-(méthylcarbamoyl)-3-(4-nonylbenzylamino)cyclopentane-carboxylique (isomère A)
le chlorhydrate de l'acide 1-(méthylcarbamoyl)-3-(4-nonylbenzylamino)cyclopentane-carboxylique (isomère B)
le chlorhydrate de l'acide 3-(méthyl-4-nonylbenzylamino)-cyclopentane-1,1-dicarboxylique
le chlorhydrate de l'acide 3-[4-(4-phénylsulfanylbutoxy)benzylamino]-cyclopentane-1,1-dicarboxylique
le chlorhydrate de l'acide 3-{4-[4-(4-éthoxy-3-chlorophényl)butoxy]-benzylamino} cyclopentane-1,1-dicarboxylique
le chlorhydrate de l'acide 2-méthyl-2-[4-(4-nonylbenzylamino)but-2-ynyl]-malonique
le chlorhydrate de l'acide 2-[4-(3-chloro-4-nonylbenzylamino)but-2-ynyl]-2-méthylmalonique
le chlorhydrate de l'acide 2-méthyl-2-[4-(4-octylbenzylamino)but-2-ynyl]-malonique
le chlorhydrate de l'acide 2-[4-(4-décylbenzylamino)but-2-ynyl]-2-méthylmalonique
le chlorhydrate de l'acide 2-[3-fluoro-4-(4-hexylphényl)benzylamino]but-2-ynyl]-2-méthylmalonique
ainsi que leurs énantiomères, diastéréo-isomères, mélanges de ceux-ci, formes libres et sels pharmaceutiquement acceptables, hydrates, solvates.

14. Composé selon l'une quelconque des revendications précédentes, choisi dans le groupe constitué par :
l'acide 3-(4-nonylbenzylamino)cydopentane-1,1-dicarboxylique
l'acide 3-(4-décylbenzylamino)cyclopentane-1,1-dicarboxylique
ainsi que leurs énantiomères, diastéréo-isomères, mélanges de ceux-ci, formes libres et sels pharmaceutiquement acceptables, hydrates, solvates.

15. Procédé de préparation d'un composé selon l'une quelconque des revendications précédentes comprenant l'étape consistant à faire réagir un composé de formule (III) : 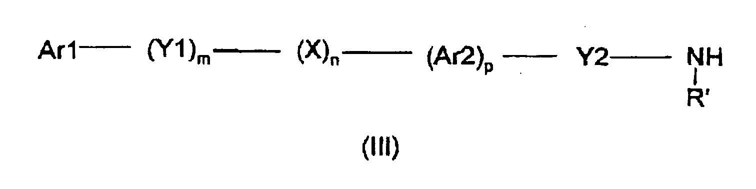 avec un composé de formule (IV) :
O=(Y3')_{q}- Z (CO₂R") (COR'") (IV)
où Ar1, Y1, X, Ar2, Y2, R', Z, R", R"', m, n, p, q sont définis comme dans la formule (I), à condition que lorsque q = 0, la fonction céto soit fixée à un atome de carbone de Z, et lorsque q = 1, Y3' soit tel que -CH2-Y3'- correspond à Y3 tel que défini dans la formule (I).

16. Procédé selon la revendication 15, où ladite réaction est réalisée dans des conditions d'amination réductrice.

17. Procédé selon la revendication 15 ou 16, où ladite réaction de couplage est suivie d'autres modifications de Ar1, Y1, X, Ar2, Y2, R', Z, R", R"' du composé de formule (I) obtenu de manière à obtenir le composé souhaité de formule (I).

18. Procédé selon l'une quelconque des revendications 15 à 17, qui comprend en outre l'étape supplémentaire consistant à isoler le composé de formule (I).

19. Composition pharmaceutique comprenant un composé de formule (I) selon l'une quelconque des revendications 1 à 14 avec un excipient ou un véhicule pharmaceutiquement acceptable.

20. Utilisation d'un composé de formule (I) : 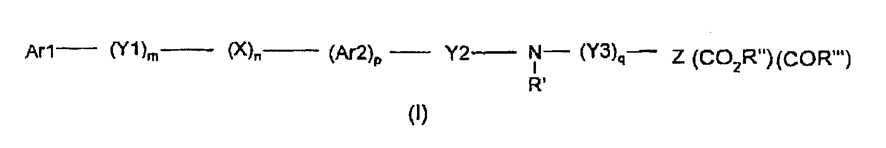 où
Ar1 représente un groupe aryle éventuellement substitué par 1 jusqu'à 5 groupes R,
où, chaque R, identique ou différent, est choisi dans le groupe constitué d'un atome d'halogène, d'un groupe -alkyle, -Oalkyle, -OH, -COOR7, -CONR7R8, -alkyl-Hal, -Oalkyl-Hal, NO₂, -CN, -NR7R8, -alkylaryle, -aryle, -S(O)₁R7, - alcényle, -Si(alkyle)₃ ;
où R7 et R8, identiques ou différents, sont choisis dans le groupe constitué de H ; un groupe alkyle éventuellement substitué par un atome d'halogène, un groupe polyfluoroalkyle, aryle ; hétéroaryle ;
ou R7 et R8 forment ensemble avec l'atome N auquel ils sont fixés un hétérocycle ;
1 vaut 0, 1 ou 2 ;
Y1 représente une chaîne -alkyl-, éventuellement substituée par un ou plusieurs R tel que défini ci-dessus, et comprenant éventuellement une ou plusieurs insaturation(s) et/ou un ou plusieurs hétéroatome(s) et/ou un résidu choisi dans le groupe constitué de -alcén-, -alcyn-, -CH=N-, -N=CH-, -CH=N-O-, -O-N=CH-, -C(=O)-, -C(=O)(OR7)-, -C(=O)(NR7)-, -N=N-, -S(O)_{I}- ;
m vaut 0 ou 1 ;
X représente un hétéroatome ;
n vaut 0 ou 1 ;
-Ar2- représente un groupe -aryl- éventuellement substitué par un ou plusieurs groupes R tel que défini ci-dessus ou bien où 2 R peuvent former ensemble avec les atomes auxquels ils sont fixés un cycle fusionné cyclique, aryle ou hétéroaryle ;
p vaut 0 ou 1;
-Y2- représente une chaîne -alkyl-, éventuellement substituée par un ou plusieurs R tel que défini ci-dessus, et comprenant éventuellement une ou plusieurs insaturation(s) ou un ou plusieurs hétéroatome(s) et/ou un résidu choisi dans le groupe constitué de -alcén-, -alcyn-, -CH=N-, -N=CH-, -CH=N-O-, -ON=CH-, -C(=O)-, -C(=O)O-, -C(=O)(NR7)-, -N=N-, -S(O)_{I}- ;
R' représente un atome d'hydrogène, ou une chaîne -alkyl-, éventuellement substituée par un ou plusieurs R tel que défini ci-dessus, et comprenant éventuellement une ou plusieurs insaturation(s) ou un ou plusieurs hétéroatome(s) et/ou un résidu choisi dans le groupe constitué de -alcén-, -alcyn-, -CH=N-, - N=CH-, -CH=N-O-, -O-N=CH-, -C(=O)-, -C(=O)O-, -C(=O)(NR7)-, -N=N-, - S(O)_{I}- ;
-Y3- représente une chaîne -alkyl-, éventuellement substituée par un ou plusieurs R tel que défini ci-dessus, et comprenant éventuellement une ou plusieurs insaturation(s) ou un ou plusieurs hétéroatome(s) et/ou un résidu choisi dans le groupe constitué de -alcén-, -alcyn-, -CH=N-, -N=CH-, -CH=N-O-, -ON=CH-, -C(=O)-, -C(=O)O-, -C(=O)(NR7)-, -N=N-, -S(O)_{I}-;
q vaut 0 ou 1;
Z représente un groupe -cycloalkyl< ou un groupe -hétérocyclique<, - aryl<, -hétéroaryl< ou un groupe -CH< ou un groupe -C(alkyl)<, ou R' et Z forment ensemble avec l'atome N auquel ils sont fixés un groupe hétérocyclique ou hétéroaryle ;
où (CO₂R") et (COR"') peuvent être fixés au même atome ou à deux atomes adjacents ;
-R" représente un atome d'hydrogène ou une chaîne alkyle, éventuellement substituée par un ou plusieurs R tel que défini ci-dessus, et comprenant éventuellement une ou plusieurs insaturation(s) ;
-R"' représente -OH, -Oalkyle, H, -NR7OR8, -NR7R8, des acides aminés naturels ou synthétiques, où R7 et R8 sont définis comme ci-dessus ;
où R" et R"' forment ensemble un cycle avec le(s) atome(s) au(x)quel(s) ils sont fixés pour former un groupe bis-carbonyle intramoléculaire cyclique, y compris des dérivés de l'acide de Meldrum ;
ainsi que leurs énantiomères, diastéréo-isomères, mélanges de ceux-ci, formes libres et sels pharmaceutiquement acceptables, hydrates, solvates
pour la préparation d'un médicament agissant en tant qu'agoniste au niveau des récepteurs S1P1 humains de manière sélective, pour une administration à un patient en ayant besoin.

21. Utilisation selon la revendication 20, où le composé de formule (I) est défini selon l'une quelconque des revendications 1 à 14.

22. Utilisation selon la revendication 20 ou 21, où ledit médicament agit en tant qu'agoniste au niveau des récepteurs S1P1 et S1P2 humains mais est essentiellement inactif au niveau des récepteurs S1P3.

23. Utilisation selon l'une quelconque des revendications 20, 21 ou 22, où ledit médicament est approprié pour diminuer les lymphocytes circulants dans le sang chez un patient en ayant besoin.

24. Utilisation selon l'une quelconque des revendications 20 à 22, où ledit médicament est destiné au traitement et/ou à la prévention d'affections inflammatoires, de la polyarthrite rhumatoïde, de l'asthme, du psoriasis, de la myocardite, de la dermite atopique, de leucémies lymphocytaires, de lymphomes, de la sclérose en plaques, du lupus érythémateux, de maladies intestinales inflammatoires.

25. Utilisation selon l'une quelconque des revendications 20 à 22, où ledit médicament est destiné au traitement et/ou à la prévention d'un rejet de greffe de tissu.
